# EUROPEAN PATENT APPLICATION

(11) **EP 4 349 329 A1**
(43) Date of publication of application: **10.04.2024**
(21) Application number: 22816460.4
(22) Date of filing: 01.06.2022
(51) Int. Cl.: A61K 31/165, A61P 29/00, A61P 35/00, C07C 237/20

(54) **BENZYLOXY-BENZYLAMINYL AMINO ACID DERIVATIVE FOR ADJUSTING TSLP/TSLPR SIGNAL TRANSMISSION**

(30) Priority: 01.06.2021 KR 20210071116
(71) Applicant: Amtixbio Co., Ltd., Seoul 01411 (KR)
(72) Inventor: LEE, Jongseung, Seoul 01738 (KR); LEE, Han-Seung, Hanam-si, Gyeonggi-do 12925 (KR); SEO, Kyoung Hee, Hanam-si, Gyeonggi-do 12925 (KR); KIM, Dahee, Hanam-si, Gyeonggi-do 12925 (KR); LEE, Dong-Gi, Hanam-si, Gyeonggi-do 12925 (KR)
(74) Representative: Bringer IP
(86) International application number: PCT/KR2022/007797
(87) International publication number: WO 2022/255801

(57) **Abstract**

The present invention provides a pharmaceutical composition, comprising a novel benzyloxybenzylamine amino acid derivative, its salt, and/or solvate, as an active ingredient, that inhibits intracellular signaling pathway mediated by cytokine thymic stromal lymphopoietin (TSLP), TSLP receptor (TSLPR) and IL-7Rα. The composition can be used for the prevention, treatment, or prevention and treatment of cancer, inflammatory diseases, itching, allergies, and/or immune disorders.

## Description

### Technical Field

This invention relates to a benzyloxybenzylamine amino acid derivative and a pharmaceutical composition comprising it as an active ingredient. In particular, this invention pertains to a pharmaceutical composition for treating inflammatory disease, which regulates intracellular signaling of Thymic Stromal Lymphopoietin (TSLP).

### Background Art

Cytokines play a crucial role in the immune system, including cellular-mediated immunity and allergic reactions. Cytokines can be categorized into proinflammatory cytokines and anti-inflammatory cytokines. They are primarily produced by T lymphocytes, and the antigen-specific receptors present on the surface of T lymphocytes recognize external pathogens. T lymphocytes are classified into CD4 and CD8 based on the type of cell surface molecules. T lymphocytes expressing CD4 generate the most cytokines and are known as helper T cells. Furthermore, helper T cells are subdivided into Th1, which produces Th1-type cytokines, and Th2, which produces Th2-type cytokines. Th1-type cytokines, such as interferon-gamma, primarily induce inflammatory responses to maintain immune reactions for the removal of intracellular foreign pathogens. On the other hand, Th2-type cytokines include interleukin-4, interleukin-5, interleukin-13, and interleukin-10 etc., which are associated with promoting IgE and eosinophilic responses in atopic conditions.

Thymic stromal lymphopoietin (TSLP) is generated by skin keratinocytes or epithelial cells in the lungs and intestines in response to stimuli by microorganisms, such as virus, bacteria, fungus, protease allergens, inflammatory cytokines, chemical substances, and other allergens. It is produced by skin keratinocytes or epithelial cells in the lungs and intestines. TSLP performs its biological functions by initially binding to its receptor, TSLP receptor (TSLPR). Subsequently, it forms a heterodimeric complex with interleukin-7 receptor subunit alpha (IL7Rα), activating a signaling cascade. This heterodimeric complex polarizes dendritic cells, inducing type 2 inflammatory responses and acting as a master regulator for Th2 immune responses on the surfaces of the skin, gut, and airways. When TSLP forms a complex with TSLPR and IL-7Rα, it induces cross-phosphorylation of JAK1 and JAK2, followed by the activation of STATS (signal transducer and activator of transcription).

TSLP is known to be secreted not only by epithelial cells and keratinocytes but also by immune cells such as mast cells, airway smooth muscle cells, fibroblasts, or dendritic cells, regulating immune responses. The expression of TSLP in the skin has been reported to be associated with various allergic conditions (Steven F. Ziegler et al., J Allergy Clin Immunol, 2012; 130: 845-52). Furthermore, TSLP has been detected in bronchoalveolar lavage fluid from asthma patients, and a correlation has been reported that there is correlation between increased expression of TSLP in airway epithelial cells and the severity of the disease. The secretion of TSLP from skin cells has been shown to exacerbate allergic inflammatory responses upon antigen exposure and promote the onset of asthma (Juan et al., Journal of Investigative Dermatology, 2012). TSLP receptor (TSLPR) is primarily expressed in hematopoietic cells, with dendritic cells (DC) exhibiting the highest expression.

TSLP plays a role in the communication between tissue cells and immune cells, as well as in the interaction of innate and adaptive immune systems. Therefore, TSLP and its signaling are known to be associated with conditions such as atopic dermatitis (AD), psoriasis, asthma, allergic rhinitis (AR), and eosinophilic esophagitis (EoE). In addition, TSLP signaling has been reported to be related to other immune-mediated disorders, including solid cancers (such as breast, colorectal, and pancreatic cancers) and hematologic cancers (such as B-cell acute lymphoblastic leukemia (B-ALL)). Furthermore, TSLP is recognized as a significant pruritogen, contributing to itchiness. Tezepelumab, a drug targeting TSLP, is under development as a human monoclonal antibody for the treatment of asthma and atopic dermatitis (TSLP: from allergy to cancer, Jonathan Corren & Steven F. Ziegler, Nature Immunology volume 20, 1603 - 1609 (2019)). However, biological drugs like antibodies have limitations in tissue penetration, face challenges in oral development, and may not cost effective.

The inventors have endeavored to develop a drug for the treatment of inflammation, Th2 immune-mediated disorders, and/or cancer by regulating TSLP signaling, and as a result, completed the present invention.

### Technical Problem

The present invention aims to provide a pharmaceutical composition for regulating TSLP/TSLPR signaling, comprising a novel benzyloxybenzylamine amino acid derivative as an active ingredient.

Another object of the present invention is to provide a pharmaceutical composition for the prevention, treatment, or prevention and treatment of inflammation, immune disorders, and/or cancer, comprising a novel benzyloxybenzylamine amino acid derivative as an active ingredient.

Yet another object of the present invention is to provide a pharmaceutical composition for the prevention or treatment, and prevention and treatment, of itching, comprising a novel benzyloxybenzylamine amino acid derivative as an active ingredient.

Still yet another object of the present invention is to provide a pharmaceutical composition for the prevention or treatment, and prevention and treatment, of allergic diseases, comprising a novel benzyloxybenzylamine amino acid derivative as an active ingredient.

In addition, still yet another object of the present invention is to provide a method for treating cancer, inflammatory conditions, itching, allergies, and/or immune disorders by administering a pharmaceutical composition comprising a novel benzyloxybenzylamine amino acid derivative as an active ingredient.

### Solution to Problem

In order to achieve the above objects, the present invention provides a benzyloxybenzylamine amino acid derivative represented by the following Formula 1, a pharmaceutically acceptable salt, and/or a solvate thereof. 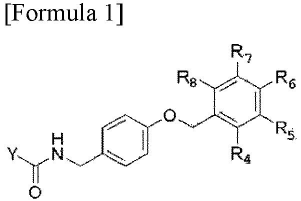 in the Formula 1, R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and Y is selected from the following Formulas 2 to 4: 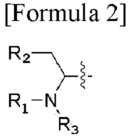 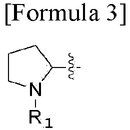 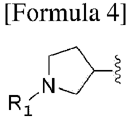
wherein R₁ and R₃ are the same or different from each other, and are each independently hydrogen or C₁₋₇ alkyl, and
R₂ is C₁₋₇ alkyl.

In the present invention, the halogen may be selected from fluoro, chloro, bromo, and iodo, and the C₁₋₆ alkyl may be a linear, branched, or cyclic alkyl and may be selected from methyl, ethyl, propyl, isopropyl, cyclopropyl, butyl, isobutyl, sec-butyl, t-butyl, pentyl, hexyl, heptyl, and octyl.

The C₁₋₇ alkyloxy group may be selected from methoxy, ethoxy, propoxy, butoxy, pentoxy, hexyloxy, heptyloxy, and octyloxy.

The halogenated C₁₋₇ alkyl may be selected from difluoromethyl, trifluoromethyl, difluoroethyl, trifluoroethyl, trifluoropropyl, trifluoropentyl, trifluorohexyl, and trifluoroheptyl, and the halogenated C₁₋₇ alkyloxy may be selected from difluoromethyloxy, trifluoromethyloxy, difluoroethyloxy, trifluoroethyloxy, trifluoropropyloxy, trifluoropentyloxy, trifluorohexyloxy, and trifluoroheptyloxy.

According to the present invention, in the Formula 1, in a case where any one of R₄, R₅, R₆, R₇, and R₈ is C₁₋₇ alkyl, hydroxy, halogen, halogenated C₁₋₇ alkyl, C₁₋₇ alkyloxy, or halogenated C₁₋₇ alkyloxy, the other four may be hydrogen atoms.

The compound represented by the Formula 1 may be specifically, for example, any one selected from the following compounds, or a racemic mixture of R-form and S-form thereof:
*(R)*/*(S)*-1-((4-((2-(fluorobenzyl)oxy)benzyl)amino)-1-oxobutane-2-aminium chloride (Compound 17);
*(R)*/*(S)*-1-((4-((3-(fluorobenzyl)oxy)benzyl)amino)-1-oxobutane-2-aminium chloride (Compound 18);
*(R)*/*(S)*-1-((4-((4-(fluorobenzyl)oxy)benzyl)amino)-1-oxobutane-2-aminium chloride (Compound 19);
*(R)*/*(S)*-1-oxo-1-((4-((2-(trifluoromethyl)benzyl)oxy)benzyl)amino)butane-2-aminium chloride (Compound 20);
*(R)*/*(S)*-1-oxo-1-((4-((3-(trifluoromethyl)benzyl)oxy)benzyl)amino)butane-2-aminium chloride (Compound 21);
*(R)*/*(S)*-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)*-1-((4-((3-fluorobenzyl)oxy)benzyl)amino)-1-oxopentane-2-aminium chloride (Compound 25);
*(R)*/*(S)*-1-((4-((4-(trifluorobenzyl)oxy)benzyl)amino)-1-oxopentane-2-aminium chloride (Compound 26);
*(R)*/*(S)*-2-(methylamino)-N-(4-((4-trifluoromethyl)benzyl)oxy)benzyl)butanamide (Compound 27);
*(R)*/*(S)-*N-(4-((3-chlorobenzyl)oxy)benzyl)-2-(methylamino)butanamide (Compound 28);
*(R)*/*(S)*-N-(4-((4-chlorobenzyl)oxy)benzyl)-2-(methylamino)butanamide (Compound 29);
*(R)*/*(S)*-2-(methylamino)-N-(4-((4-trifluoromethyl)benzyl)oxy)benzyl)pentanamide (Compound 30);
*(R)*/*(S)*-2-(methylamino)-N-(4-((4-trifluoromethyl)benzyl)oxy)benzyl)pentanamide (Compound 31);
*(R)*/*(S)*-tert-butyl-2-((4-((3-fluorobenzyl)oxy)benzyl)carbamoyl)pyrrolidine-1-carboxylate (Compound 34);
*(R)*/*(S)*-tert-butyl-2-((4-((4-fluorobenzyl)oxy)benzyl)carbamoyl)pyrrolidine-1-carboxylate (Compound 35);
*(R)*/*(S)*-tert-butyl-2-((4-((4-(trifluoromethyl)benzyl)oxy)benzyl)carbamoyl)pyrrolidine-1-carboxylate (Compound 36);
*(R)*/*(S)*-tert-butyl-2-((4-((4-chlorobenzyl)oxy)benzyl)carbamoyl)pyrrolidine-1-carboxylate (Compound 37);
*(R)*/*(S)*-N-(4-((3-fluorobenzyl)oxy)benzyl)pyrrolidine-2-carboxamide hydrochloride (Compound 38);
*(R)*/*(S)*-N-(4-((4-fluorobenzyl)oxy)benzyl)pyrrolidine-2-carboxamide hydrochloride (Compound 39);
*(R)*/*(S)*-N-(4-((4-(trifluoromethyl)benzyl)oxy)benzyl)pyrrolidine-2-carboxamide hydrochloride (Compound 40);
*(R)*/*(S)*-N-(4-((4-chlorobenzyl)oxy)benzyl)pyrrolidine-2-carboxamide hydrochloride (Compound 41);
*(R)*/*(S)-*1-methyl-N-(4-((3-fluorobenzyl)oxy)benzyl)pyrrolidine-2-carboxamide (Compound 42);
*(R)*/*(S)*-1-methyl-N-(4-((4-(trifluoromethyl)benzyl)oxy)benzyl)pyrrolidine-2-carboxamide (Compound 43);
*(R)*/*(S)*-1-methyl-N-(4-((4-chlorobenzyl)oxy)benzyl)pyrrolidine-2-carboxamide (Compound 44);
*(R)*/*(S)*-tert-butyl-3-((4((3-fluorobenzyl)oxy)benzyl)carbamoyl)pyrrolidine-1-carboxylate (Compound 46);
*(R)*/*(S)*-tert-butyl-3-((4((4-(trifluoromethyl)benzyl)oxy)benzyl)carbamoyl)pyrrolidine-1-carboxylate (Compound 47);
*(R)*/*(S)*-tert-butyl-3-((4((4-chlorobenzyl)oxy)benzyl)carbamoyl)pyrrolidine-1-carboxylate (Compound 48);
*(R)*/*(S)*-2-((4-((3-fluorobenzyl)oxy)benzyl)carbamoyl)pyrrolidi-1-nium chloride (Compound 49);
*(R)*/*(S)*-2-((4-((4-(trifluoromethyl)benzyl)oxy)benzyl)carbamoyl)pyrrolidi-1-nium chloride (Compound 50);
*(R)*/*(S)*-2-((4-((4-chlorobenzyl)oxy)benzyl)carbamoyl)pyrrolidi-1-nium chloride (Compound 51);
*(R)*/*(S)*-1-methyl-N-(4-((3-fluorobenzyl)oxy)benzyl)pyrrolidine-3-carboxamide (Compound 52);
*(R)*/*(S)*-1-methyl-N-(4-((4-(trifluoromethyl)benzyl)oxy)benzyl)pyrrolidine-3-carboxamide (Compound 53);
*(R)*/*(S)*-1-methyl-N-(4-((4-chlorobenzyl)oxy)benzyl)pyrrolidine-3-carboxamide (Compound 54);
*(R)*/*(S)*-tert-butyl(1-((4-((3,4-chlorobenzyl)oxy)benzyl)amino)-1-oxobuta-2-nyl)carbamate (Compound 55);
*(R)*/*(S)*-tert-butyl(1-oxo-1-((4-((4-(trifluoromethyl)benzyl)oxy)benzyl)amino)penta-2-nyl)carbamate (Compound 56);
*(R)*/*(S)*-1-((4-((3,4-dichlorobenzyl)oxy)benzyl)amino)-1-oxobutane-2-aminium chloride (Compound 57);
*(R)*/*(S)-1-((4-((3,4-dichlorobenzyl)oxy)benzyl)amino)-1-oxopentane-2-aminium* chloride (Compound 58);
*(R)*/*(S)*-2-(dimethylamino)-N-(4-((4-trifluoromethyl)benzyl)oxy)benzyl)butanamide (Compound 59);
*(R)*/*(S)-*N-(4-((3,4-dichlorobenzyl)oxy)benzyl)-2-(dimethylamino)butanamide (Compound 60);
*(R)*/*(S)*-N-(4-((3,4-dichlorobenzyl)oxy)benzyl)-2-(dimethylamino)butanamide (Compound 61);
*(R)*/*(S)*-2-(ethylamino)-N-(4-((4-(trifluoromethyl)benzyl)oxy)benzyl)butanamide (Compound 62);
*(R)*/*(S)*-N-(4-((3,4-dichlorobenzyl)oxy)benzyl-2-(ethylamino)butanamide (Compound 63);
*(R)*/*(S)*-2-(ethylamino)-N-(4-((4-(trifluoromethyl)benzyl)oxy)benzyl)pentanamide (Compound 64);
*(R)*/*(S)*-N-(4-((3,4-dichlorobenzyl)oxy)benzyl-2-(ethylamino)pentanamide (Compound 65);
*(R)*/*(S)*-2-(diethylamino)-N-(4-((4-(trifluoromethyl)benzyl)oxy)benzyl)butanamide (Compound 66);
*(R)*/*(S)*-N-(4-((3,4-dichlorobenzyl)oxy)benzyl-2-(diethylamino)butanamide (Compound 67);
*(R)*/*(S)*-2-(diethylamino)-N-(4-((4-(trifluoromethyl)benzyl)oxy)benzyl)pentanamide (Compound 68);
*(R)*/*(S)*-N-(4-((3,4-dichlorobenzyl)oxy)benzyl-2-(diethylamino)pentanamide (Compound 69).

In the present invention, the pharmaceutically acceptable salt is not particularly limited as long as it is conventionally used in the art. Specific examples thereof include, but are not limited to, those capable of forming a salt with an inorganic acid such as hydrochloric acid, bromic acid, sulfonic acid, amidosulfuric acid, phosphoric acid or nitric acid, or an organic acid such as carboxylic acid or sulfonic acid.

In addition, examples of the carboxylic acid may include acetic acid, maleic acid, fumaric acid, malic acid, citric acid, tartaric acid, lactic acid, benzoic acid, succinic acid, propionic acid, glycolic acid, stearic acid, lactic acid, and the like, and examples of the sulfonic acid may include methansulfonic acid, ethanesulfonic acid, benzenesulfonic acid, toluenesulfonic acid, and naphthalenedisulfonic acid. However, types of the carboxylic acid and sulfonic acid are not limited to the above-mentioned compounds.

The pharmaceutical composition comprising the amino acid derivative represented by the Formula 1 as a pharmaceutically active ingredient can be useful for the prevention or treatment of inflammatory diseases such as atopic dermatitis, psoriasis, asthma, inflammatory arthritis, inflammatory bowel diseases, immune disorders, allergies, and cancer. In addition, these compositions can be formulated for co-administration with existing drugs as pharmaceutical compositions for combined therapy.

The benzyloxybenzyl amino acid derivative according to the present invention can be prepared by a method comprising the following steps, as described in the applicant's previous patent (Korean Patent Gazette 10-2099980):
1) protecting an amino group of an amino acid selected from the following Formulas 5 to 7 with a protecting group;
2) reacting the amino acid having a protected amine group with a benzylamine derivative, to prepare a benzyloxybenzylamine amino acid derivative in which the amino group of the amino acid is protected with the protecting group; and
3) removing the amine protecting group. 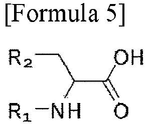 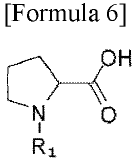 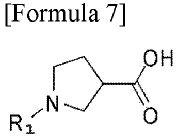 in the Formulas 5 to 7,
   R₁ is hydrogen or C₁₋₇ alkyl,
   R₂ is C₁₋₇ alkyl.

First, the amine group of the amino acid selected from the Formulas 5 to 7 is protected with an amine protecting group. For the amine protecting group, any group may be used as long as it is conventionally used for amine protection.

Next, the amino acid having a protected amine group is reacted with a benzylamine derivative to prepare a benzyloxybenzylamine amino acid derivative in which the amine group of the amino acid is protected with the protecting group. The reaction may be performed in one step or in two steps. In a case where the reaction is performed in one step, the amino acid having a protected amine group may be reacted with a benzyloxybenzylamine derivative. In a case where the reaction is performed in two steps, the amino acid having a protected amine group may be reacted with 4-hydroxybenzylamine to prepare a 4-hydroxybenzylamine amino acid derivative in which the amine group of the amino acid is protected with the protecting group, and then the resultant may be reacted with a benzyl bromide derivative.

The amine protecting group may be removed using a conventional amine removal method. In the present invention, treatment with perchloric acid was used.

In the benzyloxybenzylamine amino acid derivative according to the present invention, the amine of the amino acid residue may be primary, secondary, or tertiary. In a case where the amine is secondary or tertiary, an alkylation reaction may be performed to introduce an alkyl group.

According to the present invention, the benzyloxybenzylamine amino acid derivative or a pharmaceutically acceptable salt thereof may be formulated with a conventional formulation method by being mixed with a conventional carrier, adjuvant, or diluent, and may be prepared in a form suitable for oral administration or parenteral administration.

Examples of the carrier, adjuvant, or diluent may include lactose, dextrose, sucrose, sorbitol, mannitol, xylitol, erythritol, maltitol, starch, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate, and mineral oil.

In addition, in a case of being made into a preparation, conventional fillers, extenders, binders, wetting agents, disintegrants, surfactants, and the like may be further contained, and lubricants such as magnesium stearate and talc may be further added.

For the oral administration, the formulation may be prepared in the form of tablets, capsules, solutions, syrups, suspensions, or the like. For the parenteral administration, the formulation may be prepared in the form of injectable formulations for intraperitoneal, subcutaneous, or intramuscular injections. It can be manufactured in the form of an inhalation preparation used in a nebulizer or inhaler. The formulation may be manufactured in the form of a patch-type preparation such as a microneedle, or an external preparation such as topical application.

According to an embodiment of the present invention, the effective daily dose of the benzyloxybenzylamine amino acid derivative of Formula 1 or a pharmaceutically acceptable salt thereof in the pharmaceutical composition is 0.01 to 2,000 mg/day on an adult basis. However, the dose may vary depending on the patient's age, body weight, sex, dosage form, health condition and severity of disease, and may be administered once a day or divided into several times a day at a certain time interval according to the judgment of a doctor or a pharmacist.

### Advantageous Effects of Invention

The pharmaceutical composition of the present invention can be used for the development of drugs that effectively prevent and treat inflammatory diseases, immune disorders, allergies, itching, and/or cancer by inhibiting TSLP/TSLPR signaling.

### Brief Description of Drawings

Figures 1 to 3 demonstrate the inhibitory activity of the compounds of the present invention against IL-6, IL-4, IL-13, and TSLP.
Figures 4 and 5 present the inhibition of STATS phosphorylation by the compounds of the present invention in HEK293 cells.
Figure 6 illustrates the SCOARD index confirmed in a DNCB-induced inflammatory mouse model.
Figure 7 shows the H&E staining results of skin tissues obtained from DNCB-induced atopic dermatitis mice.
Figure 8 reveals the measurement of IgG and TSLP in the serum of DNCB-induced atopic dermatitis mice (*, *P* < 0.05; **, *P* < 0.01; ***, *P* < 0.001).
Figure 9 presents the analysis of TNF-α, IL-1β, CCL22/MDC, IL-4, MMP-12, and S100A9 in the skin tissues of DNCB-induced atopic dermatitis mice.
Figure 10 shows the anti-itching effect of the compounds of the present invention in DNFB-induced atopic dermatitis mice.
Figure 11 shows results of the observation of inflammation scores in house dust mite (HDM)-induced atopic dermatitis mice.
Figure 12 illustrates a graph presenting the skin lesion scores in HDM-induced atopic dermatitis mice.
Figure 13 illustrates an analysis result of the IgE levels in the serum of HDM-induced atopic dermatitis mice.
Figure 14 presents H&E staining images confirming skin tissue changes and measurements of eosinophils and skin thickness in HDM-induced atopic dermatitis mice.
Figure 15 shows the results of FACS analysis of skin-specific T cell fraction in HDM atopic dermatitis mouse skin tissue.
Figure 16 exhibits the results of FACS analysis of skin-specific T-cell fractions in skin tissues isolated from patients with atopic dermatitis.

### Detailed Description of Invention

Schemes for preparing the compounds of the present invention are as follows. In the following schemes, numbers which are not used together with alphabetic letters represent compound numbers prepared in the present invention. That is, 1 represents Compound 1:

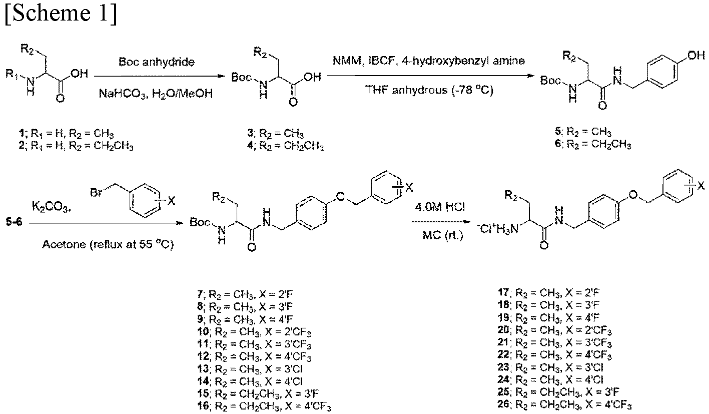

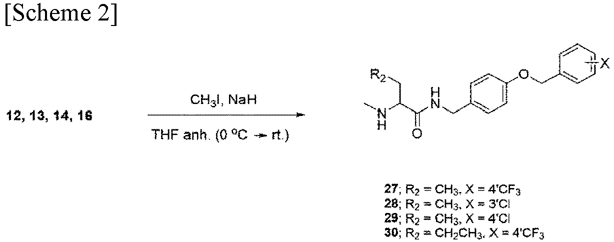

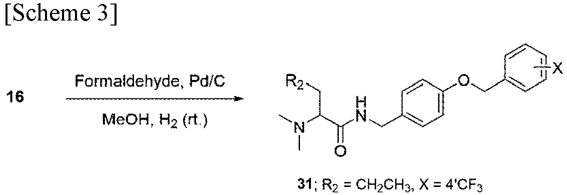

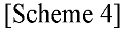

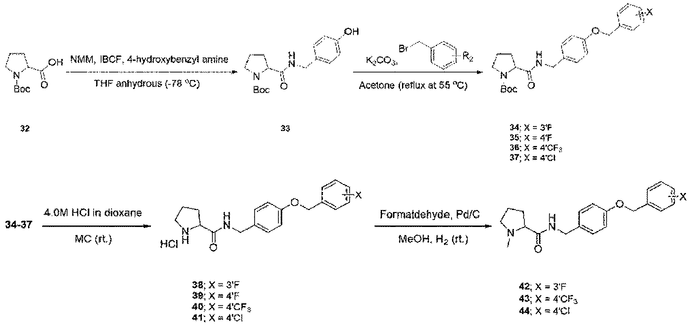

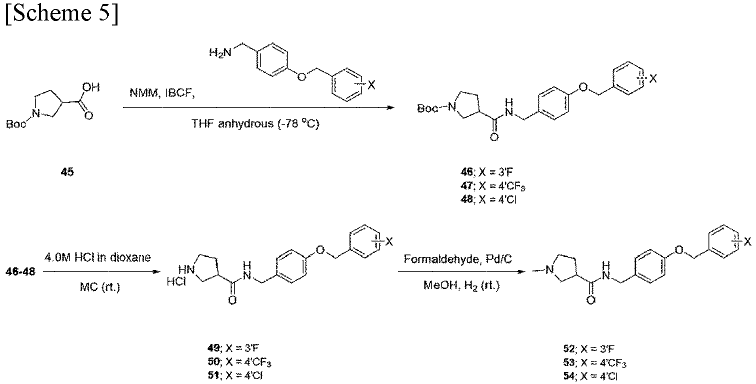

Synthesis examples for preparing the compounds of the present invention are as follows:

### Synthesis Example 1: Protection of amine group of amino acid

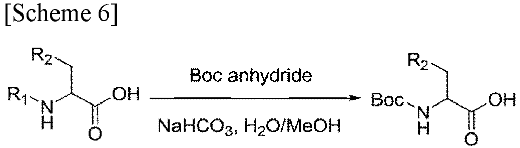

An amino acid (1.0 eq.), Boc anhydride (1.5 eq.), and sodium bicarbonate (1.5 eq.) were dissolved in a mixed solvent of distilled water and methanol (1:1), and allowed to react at room temperature for 36 to 48 hours. After completion of the reaction, the resultant was washed twice with diethyl ether, and then the pH of the water layer was adjusted to 2 with 1.0 M hydrochloric acid. Then, extraction with ethyl acetate (EA) was performed. The obtained organic layer was dehydrated using a desiccant (sodium sulfate) and dried under reduced pressure, to obtain the desired compound, an amino acid of which an amine group had been protected with a protecting group (Boc).

### Synthesis Example 2: Binding with 4-hydroxvbenzylamine

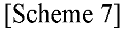

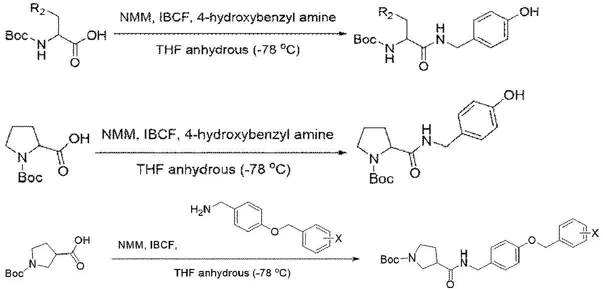

The compound (0.1 M) synthesized in the Synthesis Example 1 and N-methylmorpholine (NMM) (0.15 M, 1.5 eq.) were added to anhydrous tetrahydrofuran at a low temperature of -78°C, and the mixture was stirred for 5 minutes. Then, isobutyl chloroformate (IBCF) (0.13 M, 1.3 eq.) was added thereto, and the resultant was stirred for 5 minutes. Then, 4-hydroxybenzylamine derivative (0.12 M, 1.2 eq.) was added thereto and the resultant was stirred for 5 minutes. The temperature of the reaction mixture was raised to room temperature, and then it was identified whether the reaction proceeded while performing stirring for 30 minutes to 60 minutes. After completion of the reaction, the mixture was filtered and dried under reduced pressure, and then purified by silica gel column chromatography.

### Synthesis Example 3: Reaction for binding with benzyl bromide

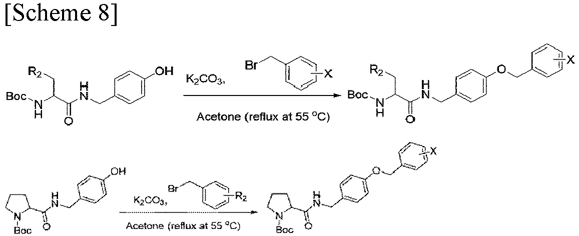

The compound (1.0 eq.) of the Synthesis Example 2, benzyl bromide (1.0 to 1.2 eq.), and potassium carbonate (4.0 eq.) were dissolved in acetone, and then it was identified whether the reaction proceeded while performing refluxing at 50°C to 60°C for 4 to 12 hours. After completion of the reaction, the reaction mixture was concentrated. Then, extraction with methylene chloride was performed, and water remaining in the organic layer was removed with a desiccant (sodium sulfate). The obtained organic layer was concentrated and then purified by silica gel column chromatography.

### Synthesis Example 4: Removal of amine protecting group

The compound obtained in the Synthesis Example 3 was dissolved in methylene chloride. Then, an excessive amount (6 to 10 eq.) of 4.0 M hydrochloric acid was added thereto and stirring was performed at room temperature. After completion of the reaction, the resulting solid was washed with ethyl acetate and then dried under vacuum to remove the amine protecting group (boc). As a result, a benzyloxybenzylamine amino acid derivative containing the desired primary amine structure was obtained.

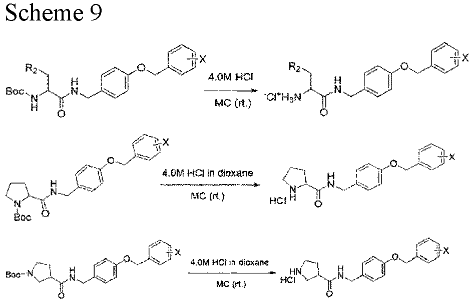

### Synthesis Example 5.1: Alkylation 1

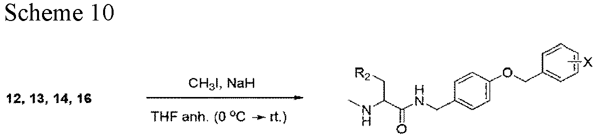

The benzyloxybenzylamine amino acid derivative of which the amino acid has a primary amine structure was subjected to alkylation, to have a secondary amine structure. Specifically, the compound (1.0 eq.) of Synthesis Example 4 and iodomethane (10 eq.) were dissolved in tetrahydrofuran, and then sodium hydride (10 eq.) was slowly added dropwise thereto at 0°C. Then, the resultant was allowed to react at room temperature for 24 hours. After completion of the reaction, the reaction mixture was extracted with methylene chloride and dehydrated with a desiccant (sodium sulfate). Then, concentration under reduced pressure was performed. The obtained concentrate was purified by silica gel column chromatography. The purified compound was dissolved in methylene chloride and then the resultant was stirred while adding 6.0 to 10.0 eq. of 4.0 M hydrochloric acid thereto. After completion of the reaction, the resultant was concentrated under reduced pressure, and then the concentrate was purified by silica gel column chromatography to obtain a benzyloxybenzylamine amino acid derivative containing a secondary amine structure.

### Synthesis Example 5.2: Alkylation 2

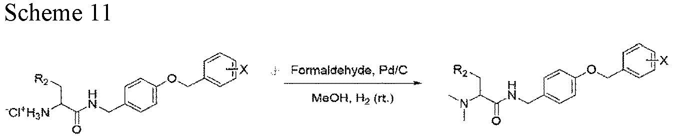

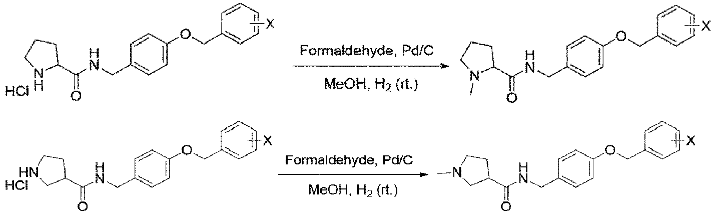

The benzyloxybenzylamine amino acid derivative of which the amino acid has a primary amine structure was subjected to alkylation, to have a tertiary amine structure. Specifically, the compound (1.0 eq.) of Synthesis Example 4 was dissolved in methanol. Then, formaldehyde (37% by weight solution) and 10% palladium catalyst were sequentially added thereto, and the resultant was allowed to react at room temperature for 18 hours. After the reaction, the catalyst was filtered off with celite, and the filtrate was evaporated under reduced pressure. The reaction mixture was recrystallized from methanol/diethyl ether to obtain the purified desired compound.

### Synthesis Example 6: General method for ethylation and diethylation of amine

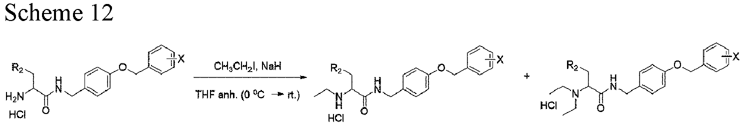

The compound (1.0 eq.) obtained in the Synthesis Example 4 and iodoethane (0.9 eq.) were dissolved in a tetrahydrofuran solvent, and sodium hydride (10 eq.) was added dropwise very slowly thereto at 0°C. The resultant was allowed to react at room temperature for 24 hours. After completion of the reaction, the resultant was diluted with a dimethyl chloride solvent and washed with distilled water. Then, water in the organic layer was dried over sodium sulfate and concentrated *in vacuo.* The obtained residue was separated and purified by chromatography using silica gel. The purified product was dissolved in a dimethyl chloride solvent, and then 4.0M hydrochloric acid (6.0 to 10.0 eq.) was added thereto while performing stirring at room temperature. Then, concentration *in vacuo* was performed. The obtained residue was separated and purified by chromatography using silica gel.

Hereinafter, the present invention will be described in more detail with reference to preferred examples. However, it will be apparent to those skilled in the art that these examples are intended to more specifically describe the present invention and the scope of the present invention is not limited thereby.

### Preparation examples

### Preparation example 1:

### (R)/(S)-2-((tert-butoxycarbonyl)amino)butanoic acid (Compound 3)

Using the above Scheme 6, Compound 1 (3.00 g, 29.1 mmol), Boc anhydride (10.00 mL, 43.6 mmol), and NaHCO₃ (3.67 g, 43.6 mmol) were reacted to synthesize Compound 3 in the form of white powder (5.89 g, 100%): Rε = 0.50 (DCM 19 : Methanol 1 and few drops of acetic acid); ¹H NMR (DMSO-d₆, 400 MHz) 12.40 (C(O)OH), 7.02 (d, J = 7.9 Hz, NH), 3.74-3.82 (m, NHCHCH₂), 1.50-1.73 (m, CH₂CH₃), 1.38 (s, Boc), 0.87 (t, J = 7.4 Hz, CH₂CH₃).

### Preparation example 2:

### (R)/(S)-2-((tert-butoxycarbonyl)amino)pentanoic acid (Compound 4)

Using the above Scheme 6, Compound 2 (3.00 g, 25.6 mmol), Boc anhydride (8.82 mL, 38.4 mmol), and NaHCO₃ (3.24 g, 38.4 mmol) were reacted to synthesize Compound 4 in the form of white powder (5.07 g, 91%): R_{f} = 0.50 (DCM 19 : Methanol 1 and few drops of acetic acid); ¹H NMR (DMSO-d₆, 400 MHz) 12.38 (C(O)OH), 7.03 (d, J = 8.0 Hz, NH), 3.81-3.89 (m, NHCHCH₂), 1.47-1.64 (m, CH₂CH₃), 1.38 (s, Boc), 0.85 (t, J = 7.4 Hz, CH₂CH₃).

### Preparation example 3:

### (R)/(S)-tert-butyl(1-((4-hydroxybenzyl)amino)-1-oxobuta-2-nyl)carbamate (Compound 5)

Using the above Scheme 7, Compound 3 (1.05 g, 5.2 mmol), NMM (0.85 mL, 7.8 mmol), IBCF (0.85 mL, 6.6 mmol), and 4-hydroxybenzylamine (0.76 g, 6.2 mmol) were reacted to synthesize Compound 5 in the form of light pink powder was synthesized (1.27 g, 80%): Rε = 0.35 (EtOAc 1 : n-hexane 1); ¹H NMR (DMSO-d₆, 400 MHz) 9.27 (s, Ar-OH), 8.15 (t, J = 5.7 Hz, C(O)NH), 7.03 (d, J = 8.1 Hz, ArH), 6.67 (d, J = 8.0 Hz, ArH), 6.78 (d, J = 8.0 Hz, Boc-NH), 4.08-4.22 (m, NHCH₂), 3.79-3.87 (m, NHCHCH₂), 1.45-1.67 (m, CH₂CH₃), 1.38 (s, Boc), 0.82 (t, J = 7.3 Hz, CH₂CH₃).

### Preparation example 4:

### (R)/(S)-tert-butyl(1-((4-hydroxybenzyl)amino)-1-oxopenta-2-nyl)carbamate (Compound 6)

Using the above Scheme 7, Compound 4 (4.50 g, 20.7 mmol), NMM (3.41 mL, 31.1 mmol), IBCF (3.41 mL, 26.3 mmol), and 4-hydroxybenzylamine (3.06 g, 24.9 mmol) were reacted to synthesize Compound 6 in the form of light pink power (4.09 g, 61%): Rε = 0.45 (EtOAc 1 : n-hexane 1); ¹H NMR (DMSO-d₆, 400 MHz) 9.27 (s, Ar-OH), 8.15 (t, J = 5.7 Hz, Boc-NH), 7.03 (d, J = 8.1 Hz, ArH), 6.67 (d, J = 8.0 Hz, ArH), 6.78 (d, J = 8.0 Hz, C(O)NH), 4.08-4.22 (m, NHCH₂), 3.79-3.87 (m, NHCHCH₂), 1.45-1.67 (m, CH₂CH₃), 1.38 (s, Boc), 0.82 (t, J = 7.3 Hz, CH₂CH₃).

### Preparation example 5:

### (R)/(S)-tert-butyl(1-((4-((2-fluorobenzyl)oxy)benzyl)amino)-1-oxobuta-2-nyl)carbamate (Compound 7)

Using the above Scheme 8, Compound 5 (0.10 g, 0.3 mmol), 2-fluorobenzyl bromide (0.07 g, 0.4 mmol), and K₂CO₃ (0.18 g, 1.3 mmol) were reacted to synthesize 7 in the form of white powder (0.11 g, 81%): Rε = 0.60 (EtOAc 1 : n-hexane 1); ¹H NMR (DMSO-d₆, 400 MHz) 8.22 (t, J = 5.9 Hz, C(O)NH), 7.50-7.57 (m, ArH), 7.38-7.45 (m, ArH), 7.15-7.28 (m, ArH), 6.93-6.98 (m, ArH), 6.78-6.83 (m, Boc-NH), 5.11 (s, OCH₂), 4.14-4.27 (m, NHCH₂), 3.80-3.87 (m, NHCHCH₂), 1.46-1.67 (m, CH₂CH₃), 1.38 (s, Boc), 0.82 (t, J = 7.4 Hz, CH₂CH₃).

### Preparation example 6:

### (R)/(S)-tert-butyl(1-((4-((3-fluorobenzyl)oxy)benzyl)amino)-1-oxobuta-2-nyl)carbamate (Compound 8)

Using the above Scheme 8, Compound 5 (0.20 g, 1.0 mmol), 3-fluorobenzyl bromide (0.27 g, 1.2 mmol), and K₂CO₃ (0.55 g, 4.0 mmol) were reacted to synthesize 8 in the forme of white power (0.16 g, 39%): R_{f} = 0.50 (EtOAc 1 : n-hexane 1); ¹H NMR (DMSO-d₆, 400 MHz) ¹H NMR (DMSO-d₆, 400 MHz) 8.21 (t, J = 5.8 Hz, C(O)NH), 7.39-7.47 (m, ArH), 7.23-7.29 (m, ArH), 7.11-7.20 (m, ArH), 6.91-6.97 (m, ArH), 6.80 (d, J = 8.0 Hz, Boc-NH), 5.11 (s, OCH₂), 4.13-4.27 (m, NHCH₂), 3.80-3.87 (m, NHCHCH₂), 1.45-1.67 (m, CH₂CH₃), 1.37 (s, Boc), 0.82 (t, J = 7.3 Hz, CH₂CH₃).

### Preparation example 7:

### (R)/(S)-tert-butyl(1-((4-((4-fluorobenzyl)oxy)benzyl)amino)-1-oxobuta-2-nyl)carbamate (Compound 9)

Using the above Scheme 8, Compound 5 (0.20 g, 0.7 mmol), 4-fluorobenzyl bromide (0.09 g, 0.7 mmol), and K₂CO₃ (0.36 g, 2.6 mmol) were reacted to synthesize 9 in the form of white powder (0.24 g, 89%): R_{f} = 0.63 (EtOAc 1 : n-hexane 1); ¹H NMR (DMSO-d₆, 400 MHz) ¹H NMR (DMSO-d₆, 400 MHz) 8.21 (t, J = 4.7 Hz, C(O)NH), 7.44-7.53 (m, ArH), 7.13-7.26 (m, ArH), 6.90-6.97 (m, ArH), 6.80 (d, J = 7.7 Hz, Boc-NH), 5.06 (s, OCH₂), 4.13-4.28 (m, NHCH₂), 3.79-3.89 (m, NHCHCH₂), 1.45-1.69 (m, CH₂CH₃), 1.37 (s, Boc), 0.82 (t, J = 7.0 Hz, CH₂CH₃).

### Preparation example 8:

### (R)/(S)-tert-butyl(1-oxo-1-((4-((2-(trifluoromethyl)benzyl)oxy)benzyl)amino)buta-2-nyl)carbamate (Compound 10)

Using the above Scheme 8, Compound 5 (0.10 g, 0.3 mmol), 2-(trifluoromethyl)benzyl bromide (0.08 g, 0.4 mmol), and K₂CO₃ (0.18 g, 1.3 mmol) were reacted to synthesize 10 in the form of white powder (0.13 g, 89%): Rε = 0.63 (EtOAc 1 : n-hexane 1); ¹H NMR (DMSO-d₆, 400 MHz) 8.22 (t, J = 5.7 Hz, C(O)NH), 7.68-7.83 (m, ArH), 7.55-7.62 (m, ArH), 7.16-7.23 (m, ArH), 6.91-6.97 (m, ArH), 6.80 (d, J = 7.9 Hz, Boc-NH), 5.21 (s, OCH₂), 4.14-4.29 (m, NHCH₂), 3.80-3.88 (m, NHCHCH₂), 1.47-1.69 (m, CH₂CH₃), 1.37 (s, Boc), 0.82 (t, J = 7.2 Hz, CH₂CH₃).

### Preparation example 9:

### (R)/(S)-tert-butyl(1-oxo-1-((4-((3-(trifluoromethyl)benzyl)oxy)benzyl)amino)buta-2-nyl)carbamate (Compound 11)

Using the above Scheme 8, Compound 5 (0.30 g, 1.0 mmol), 3-(trifluoromethyl)benzyl bromide (0.17 mL, 1.1 mmol), and K₂CO₃ (0.54 g, 3.9 mmol) were reacted to synthesize 11 in the form of white powder (0.20 g, 44%): Rε = 0.54 (EtOAc 1 : n-hexane 1) ¹H NMR (DMSO-d₆, 400 MHz) 8.22 (t, J = 5.7 Hz, C(O)NH), 7.57-7.82 (m, ArH), 7.11-7.21 (m, ArH), 6.90-6.99 (m, ArH), 6.80 (d, J = 8.1 Hz, Boc-NH), 5.20 (s, OCH₂), 4.14-4.28 (m, NHCH₂), 3.78-3.88 (m, NHCHCH₂), 1.44-1.68 (m, CH₂CH₃), 1.37 (s, Boc), 0.82 (t, J = 7.2 Hz, CH₂CH₃).

### Preparation example 10:

### (R)/(S)-tert-butyl(1-oxo-1-((4-((4-(trifluoromethyl)benzyl)oxy)benzyl)amino)propa-2-nyl)carbamate (Compound 12)

Using the above Scheme 8, Compound 5 (0.30 g, 1.0 mmol), 4-(trifluoromethyl)benzylbromide (0.17 mL, 1.1 mmol), and K₂CO₃ (0.54 g, 3.9 mmol) were reacted to synthesize 12 in the form of white powder (0.44 g, 96%): Rε = 0.24 (EtOAc 1 : n-hexane 1); ¹H NMR (DMSO-d₆, 400 MHz) 8.21 (t, J = 5.8 Hz, C(O)NH), 7.62-7.80 (m, ArH), 7.14-7.21 (m, ArH), 6.91-6.98 (m, ArH), 6.80 (d, J = 7.9 Hz, Boc-NH), 5.21 (s, OCH₂), 4.13-4.28 (m, NHCH₂), 3.80-3.87 (m, NHCHCH₂), 1.46-1.67 (m, CH₂CH₃), 1.37 (s, Boc), 0.82 (t, J = 7.3 Hz, CH₂CH₃).

### Preparation example 11:

### (R)/(S)-tert-butyl(1-((4-((3-chlorobenzyl)oxy)benzyl)amino)-1-oxobuta-2-nyl)carbamate (Compound 13)

Using the above Scheme 8, Compound 5 (0.30 g, 1.0 mmol), 3-chlorobenzyl bromide (0.22 g, 1.1 mmol), and K₂CO₃ (0.54 g, 3.9 mmol) were reacted to synthesize 13 in the form of white powder (0.38 g, 89%): Rε = 0.54 (EtOAc 1 : n-hexane 1); ¹H NMR (DMSO-d₆, 400 MHz) 8.21 (t, J = 5.9 Hz, C(O)NH), 7.48-7.51 (m, ArH), 7.35-7.45 (m, ArH), 7.15-7.19 (m, ArH), 6.91-6.96 (m, ArH), 6.80 (d, J = 8.1 Hz, Boc-NH), 5.21 (s, OCH₂), 4.13-4.27 (m, NHCH₂), 3.80-3.88 (m, NHCHCH₂), 1.45-1.67 (m, CH₂CH₃), 1.37 (s, Boc), 0.82 (t, J = 7.4 Hz, CH₂CH₃).

### Preparation example 12:

### (R)/(S)-tert-butyl(1-((4-(4-chlorobenzyl)oxy)benzyl)amino)-1-oxobuta-2-nyl)carbamate (Compound 14)

Using the above Scheme 8, Compound 5 (0.20 g, 0.7 mmol), 4-chlorobenzyl bromide (0.09 mL, 0.7 mmol), and K₂CO₃ (0.36 g, 2.6 mmol) were reacted to synthesize a compound in the form of white powder (0.28 g, 100%): Rε = 0.50 (EtOAc 1 : n-hexane 1); ¹H NMR (DMSO-d₆, 400 MHz) 8.21 (t, J = 5.9 Hz, C(O)NH), 7.42-7.52 (m, ArH), 7.13-7.20 (m, ArH), 6.90-6.95 (m, ArH), 6.80 (d, J = 8.0 Hz, Boc-NH), 5.08 (s, OCH₂), 4.13-4.27 (m, NHCH₂), 3.79-3.87 (m, NHCHCH₂), 1.45-1.68 (m, CH₂CH₃), 1.37 (s, Boc), 0.82 (t, J = 7.3 Hz, CH₂CH₃).

### Preparation example 13:

### (R)/(S)-tert-butyl(1-((4-(3-fluorobenzyl)oxy)benzyl)amino)-1-oxopenta-2-nyl)carbamate (Compound 15)

Using the above Scheme 8, Compound 6 (0.50 g, 1.6 mmol), 3-fluorobenzyl bromide (0.31 g, 1.6 mmol), and K₂CO₃ (0.86 g, 6.2 mmol) were reacted to synthesize a compound in the form of white powder (0.62 g, 92%): Rε = 0.33 (EtOAc 1 : n-hexane 3); ¹H NMR (DMSO-d₆, 400 MHz) 8.20 (t, J = 5.8 Hz, C(O)NH), 7.39-7.46 (m, ArH), 7.23-7.29 (m, ArH), 7.11-7.18 (m, ArH), 6.91-6.96 (m, ArH), 6.82 (d, J = 8.0 Hz, Boc-NH), 5.11 (s, OCH₂), 4.13-4.25 (m, NHCH₂), 3.86-3.94 (m, NHCHCH₂), 1.41-1.60 (m, CH₂CH₂CH₃), 1.37 (s, Boc), 1.20-1.32 (m, CH₂CH₂CH₃), 0.84 (t, J = 7.3 Hz, CH₂CH₂CH₃).

### Preparation example 14:

### (R)/(S)-tert-butyl(1-oxo-1-((4-((4-(trifluoromethyl)benzyl)oxy)benzyl)amino)penta-2-nyl)carbamate (Compound 16)

Using the above Scheme 8, Compound 6 (0.50 g, 1.6 mmol), 4-(trifluoromethyl)benzyl bromide (0.39 g, 1.6 mmol), and K₂CO₃ (0.86 g, 6.2 mmol) were reacted to synthesize a compound in the form of white powder (0.70 g, 94%): Rε = 0.37 (EtOAc 1 : n-hexane 3); ¹H NMR (DMSO-d₆, 400 MHz) 8.20 (t, J = 5.9 Hz, C(O)NH), 7.63-7.78 (m, ArH), 7.13-7.19 (m, ArH), 6.92-6.97 (m, ArH), 6.82 (d, J = 8.2 Hz, Boc-NH), 5.21 (s, OCH₂), 4.13-4.25 (m, NHCH₂), 3.86-3.94 (m, NHCHCH₂), 1.41-1.60 (m, CH₂CH₂CH₃), 1.37 (s, Boc), 1.18-1.32 (m, CH₂CH₂CH₃), 0.84 (t, J = 7.3 Hz, CH₂CH₂CH₃).

### Preparation example 15:

### (R)/(S)-1-((4-((2-(fluorobenzyl)oxy)benzyl)amino)-1-oxobutane-2-aminum chloride (Compound 17)

Using the above Scheme 9, Compound 7 (0.09 g, 0.22 mmol) and 4.0 M HCl in dioxane (0.38 mL, 10.8 mmol) were reacted to synthesize Compound 17 in the form of white powder (0.02 g, 22%): Rε = 0.00 (EtOAc 9 : Acetone 1); ¹H NMR (DMSO-d₆, 400 MHz) 8.85 (t, J = 5.3 Hz, C(O)NH), 8.06 (broad, NH₃⁺Cl⁻), 7.38-7.57 (m, ArH), 7.20-7.29 (m, ArH), 6.97-7.02 (m, ArH), 5.13 (s, OCH₂), 4.22-4.34 (m, NHCH₂), 3.66-3.72 (m, NH₃CHCH₂), 1.70-1.80 (m, CH₂CH₃), 0.87 (t, J = 7.5 Hz, CH₂CH₃).

### Preparation example 16:

### (R)/(S)-1-((4-((3-(fluorobenzyl)oxy)benzyl)amino)-1-oxobutane-2-aminium chloride (Compound 18)

Using the above Scheme 9, Compound 8 (0.14 g, 0.36 mmol) and 4.0 M HCl in dioxane (0.63 mL, 18.0 mmol) were reacted to synthesize Compound 18 in the form of white powder (0.10 g, 88%): Rε = 0.00 (EtOAc 9 : Acetone 1); ¹H NMR (DMSO-d₆, 400 MHz) 8.85 (t, J = 5.3 Hz, C(O)NH), 8.06 (broad, NH₃⁺Cl⁻), 7.38-7.57 (m, ArH), 7.20-7.29 (m, ArH), 6.97-7.02 (m, ArH), 5.13 (s, OCH₂), 4.22-4.34 (m, NHCH₂), 3.66-3.72 (m, NH₃CHCH₂), 1.70-1.80 (m, CH₂CH₃), 0.87 (t, J = 7.5 Hz, CH₂CH₃).

### Preparation example 17:

### (R)/(S)-1-((4-((4-(fluorobenzyl)oxy)benzyl)amino)-1-oxobutane-2-aminium chloride (Compound 19)

Using the above Scheme 9, Compound 9 (0.14 g, 0.36 mmol) and 4.0 M HCl in dioxane (0.63 mL, 18.0 mmol) were reacted to synthesize Compound 19 in the form of white powder (0.10 g, 88%): Rε = 0.00 (EtOAc 9 : Acetone 1); ¹H NMR (DMSO-d₆, 400 MHz) 8.86 (t, J = 5.7 Hz, C(O)NH), 8.07 (broad, NH₃⁺Cl⁻), 7.63-7.82 (m, ArH), 7.20-7.24 (m, ArH), 6.97-7.03 (m, ArH), 5.13 (s, OCH₂), 4.21-4.34 (m, NHCH₂), 3.66-3.72 (m, NH₃CHCH₂), 1.70-1.82 (m, CH₂CH₃), 0.86 (t, J = 7.5 Hz, CH₂CH₃).

### Preparation example 18:

### (R)/(S)-1-oxo-1-((4-((2-(trifluoromethyl)benzyl)oxy)benzyl)amino)butane-2-aminium chloride (Compound 20)

Using the above Scheme 9, Compound 10 (0.10 g, 0.21 mmol) and 4.0 M HCl in dioxane (0.37 mL, 10.7 mmol) were reacted to synthesize Compound 20 in the form of white powder (0.09 g, 99%): Rε = 0.00 (EtOAc 9 : Acetone 1); ¹H NMR (DMSO-d₆, 400 MHz) 8.88 (t, J = 5.6 Hz, C(O)NH), 8.15 (broad, NH₃⁺Cl⁻), 7.69-7.83 (m, ArH), 7.56-7.62 (m, ArH), 7.22-7.27 (m, ArH), 6.96-7.02 (m, ArH), 5.22 (s, OCH₂), 4.22-4.34 (m, NHCH₂), 3.68-3.74 (m, NH₃CHCH₂), 1.72-1.81 (m, CH₂CH₃), 0.87 (t, J = 7.4 Hz, CH₂CH₃).

### Preparation example 19:

### (R)/(S)-1-oxo-1-((4-((3-(trifluoromethyl)benzyl)oxy)benzyl)amino)butane-2-aminium chloride (Compound 21)

Using the above Scheme 9, Compound 11 (0.09 g, 0.19 mmol) and 4.0 M HCl in dioxane (0.33 mL, 9.7 mmol) were reacted to synthesize Compound 21 in the form of white powder (0.07 g, 93%): Rε = 0.00 (EtOAc 9 : Acetone 1); ¹H NMR (DMSO-d₆, 400 MHz) 8.86 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.21 (s, OCH₂), 4.22-4.34 (m, NHCH₂), 3.67-3.72 (m, NH₃CHCH₂), 1.70-1.80 (m, CH₂CH₃), 0.86 (t, J = 7.5 Hz, CH₂CH₃).

### Preparation example 20:

### (R)/(S)-1-oxo-1-((4-((4-(trifluoromethyl)benzyl)oxy)benzyl)amino)butane-2-aminium chloride (Compound 22)

Using the above Scheme 9, Compound 12 (0.40 g, 0.86 mmol) and 4.0 M HCl in dioxane (1.49 mL, 42.8 mmol) were reacted to synthesize Compound 22 in the form of white powder (0.33 g, 97%); Rε = 0.00 (EtOAc 9 : Acetone 1); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.21 (s, OCH₂), 4.22-4.34 (m, NHCH₂), 3.67-3.72 (m, NH₃CHCH₂), 1.70-1.80 (m, CH₂CH₃), 0.86 (t, J = 7.5 Hz, CH₂CH₃).

### Preparation example 21:

### (R)/(S)-1-((4-((3-(chlorobenzyl)oxy)benzyl)amino)-1-oxobutane-2-aminium chloride (Compound 23)

Using the above Scheme 9, Compound 13 (0.30 g, 0.7 mmol) and 4.0 M HCl in dioxane (1.20 mL, 34.7 mmol) were reacted to synthesize Compound 23 in the form of white powder (0.25 g, 97%): Rε = 0.00 (EtOAc 9 : Acetone 1); ¹H NMR (DMSO-d₆, 400 MHz) 8.91 (t, J = 5.7 Hz, C(O)NH), 8.20 (broad, NH₃⁺Cl⁻), 7.48-7.51 (m, ArH), 7.36-7.45 (m, ArH), 7.19-7.24 (m, ArH), 6.95-7.00 (m, ArH), 5.12 (s, OCH₂), 4.21-4.33 (m, NHCH₂), 3.69-3.74 (m, NH₃CHCH₂), 1.71-1.81 (m, CH₂CH₃), 0.86 (t, J = 7.5 Hz, CH₂CH₃).

### Preparation example 22:

### (R)/(S)-1-((4-((4-(chlorobenzyl)oxy)benzyl)amino)-1-oxobutane-2-aminium chloride (Compound 24)

Using the above Scheme 9, Compound 14 (0.10 g, 0.2 mmol) and 4.0 M HCl in dioxane (0.40 mL, 11.5 mmol) were reacted to synthesize Compound 24 in the form of white powder (0.08 g, 98%): Rε = 0.00 (EtOAc 9 : Acetone 1); ¹H NMR (DMSO-d₆, 400 MHz) 8.86 (t, J = 5.8 Hz, C(O)NH), 8.15 (broad, NH₃⁺Cl⁻), 7.44-7.46 (m, ArH), 7.19-7.24 (m, ArH), 6.95-7.00 (m, ArH), 5.10 (s, OCH₂), 4.21-4.33 (m, NHCH₂), 3.67-3.73 (m, NH₃CHCH₂), 1.70-1.80 (m, CH₂CH₃), 0.86 (t, J = 7.5 Hz, CH₂CH₃).

### Preparation example 23:

### (R)/(S)-1-((4-((3-fluorobenzyl)oxy)benzyl)amino)-1-oxopentane-2-aminium chloride (Compound 25)

Using the above Scheme 9, Compound 15 (0.55 g, 1.3 mmol) and 4.0 M HCl in dioxane (2.22 mL, 64.0 mmol) were reacted to synthesize Compound 25 in the form of white powder (0.43 g, 92%): Rε = 0.00 (EtOAc 9 : Acetone 1); ¹H NMR (DMSO-d₆, 400 MHz) 8.90 (t, J = 5.7 Hz, C(O)NH), 8.16 (broad, NH₃⁺Cl⁻), 7.40-7.47 (m, ArH), 7.11-7.30 (m, ArH), 6.96-7.01 (m, ArH), 5.13 (s, OCH₂), 4.24-4.30 (m, NHCH₂), 3.71-3.77 (m, NH₃CHCH₂), 1.65-1.73 (m, CH₂CH₂CH₃), 1.23-1.35 (m, CH₂CH₂CH₃), 0.86 (t, J = 7.3 Hz, CH₂CH₂CH₃).

### Preparation example 24:

### (R)/(S)-1-((4-((4-(trifluorobenzyl)oxy)benzyl)amino)-1-oxopentane-2-aminium chloride (Compound 26)

Using the above Scheme 8, Compound 16 (0.65 g, 1.4 mmol) and 4.0 M HCl in dioxane (2.34 mL, 67.5 mmol) were reacted to synthesize Compound 26 in the form of white powder (0.39 g, 70%): Rε = 0.00 (EtOAc 9 : Acetone 1); ¹H NMR (DMSO-d₆, 400 MHz) 8.89 (t, J = 5.7 Hz, C(O)NH), 8.15 (broad, NH₃⁺Cl⁻), 7.63-7.79 (m, ArH), 7.20-7.25 (m, ArH), 6.96-7.02 (m, ArH), 5.23 (s, OCH₂), 4.25-4.29 (m, NHCH₂), 3.71-3.77 (m, NH₃CHCH₂), 1.65-1.72 (m, CH₂CH₂CH₃), 1.23-1.35 (m, CH₂CH₂CH₃), 0.86 (t, J = 7.3 Hz, CH₂CH₂CH₃).

### Preparation example 25:

### (R)/(S)-2-(methylamino)-N-(4-((4-trifluoromethyl)benzyl)oxy)benzyl)butanamide (Compound 27)

Using the above Scheme 10, Compound 12 (0.40 g, 0.86 mmol), NaH (10 eq.), and CH₃I (10 eq.) were reacted to obtain a methylated compound, and then 4.0 M HCl in dioxane (1.49 mL, 42.8 mmol) was used to remove the Boc group, thereby synthesizing Compound 27 (0.30 g); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.18 (s, OCH₂), 4.22-4.34 (m, NHCH₂), 3.67-3.72 (m, NHCHCH₂), 2.39 (s, NHCH₃), 1.70-1.80 (m, CH₂CH₃), 0.86 (t, J = 7.5 Hz, CH₂CH₃).

### Preparation example 26:

### (R)/(S)-N-(4-((3-chlorobenzyl)oxy)benzyl)-2-(methylamino)butanamide (Compound 28)

Using the above Scheme 10, Compound 13 (1 eq.), NaH (10 eq.), and CH₃I (10 eq.) were reacted to obtain a methylated compound, and then 4.0 M HCl in dioxane (6 eq.) was used to remove the Boc group, thereby synthesizing Compound 28; ¹H NMR (DMSO-d₆, 400 MHz) 8.92 (t, J = 5.7 Hz, C(O)NH), 8.19 (broad, NH₃⁺Cl⁻), 7.40-7.49 (m, ArH), 7.11-7.32 (m, ArH), 6.96-7.03 (m, ArH), 5.14 (s, OCH₂), 4.24-4.31 (m, NHCH₂), 3.72-3.77 (m, NHCHCH₂), 2.38 (s, NHCH₃), 1.68-1.79 (m, CH₂CH₃), 0.87 (t, J = 7.3 Hz, CH₂CH₃).

### Preparation example 27:

### (R)/(S)-N-(4-((4-chlorobenzyl)oxy)benzyl)-2-(methylamino)butanamide (Compound 29)

Using the above Scheme 10, Compound 14 (1 eq.), NaH (10 eq.), and CH₃I (10 eq.) were reacted to obtain a methylated compound, and then 4.0 M HCl in dioxane (6 eq.) was used to remove the Boc group, thereby synthesizing Compound 29; ¹H NMR (DMSO-d₆, 400 MHz) 8.88 (t, J = 5.7 Hz, C(O)NH), 8.07 (broad, NH₃ ⁺Cl⁻), 7.62-7.81 (m, ArH), 7.21-7.26 (m, ArH), 6.97-7.04 (m, ArH), 5.13 (s, OCH₂), 4.22-4.31 (m, NHCH₂), 3.71-3.77 (m, NHCHCH₂), 2.40 (s, NHCH₃), 1.69-1.79 (m, CH₂CH₃), 0.88 (t, J = 7.3 Hz, CH₂CH₃).

### Preparation example 28:

### (R)/(S)-2-(methylamino)-N-(4-(4-trifluoromethyl)benzyl)oxy)benzyl)pentanamide_ (Compound 30)

Using the above Scheme 10, Compound 12 (0.40 g, 0.86 mmol), NaH (10 eq.), and CH₃I (10 eq.) were reacted to obtain a methylated compound, and then 4.0 M HCl in dioxane (1.49 mL, 42.8 mmol) was used to remove the Boc group, thereby synthesizing Compound 27 (0.30 g); ¹H NMR (DMSO-d₆, 400 MHz) 8.89 (t, J = 5.7 Hz, C(O)NH), 8.09 (broad, NH₃⁺Cl⁻), 7.61-7.82 (m, ArH), 7.20-7.25 (m, ArH), 6.98-7.03 (m, ArH), 5.15 (s, OCH₂), 4.22-4.34 (m, NHCH₂), 3.67-3.72 (m, NHCHCH₂), 2.39 (s, NHCH₃), 1.63-1.72 (m, CH₂CH₂CH₃), 1.22-1.35 (m, CH₂CH₂CH₃), 0.87 (t, J = 7.3 Hz, CH₂CH₂CH₃).

### Preparation example 29:

### (R)/(S)-2-(methylamino)-N-(4-((4-trifluoromethyl)benzyl)oxy)benzyl)pentanamide_ (Compound 31)

Using the above Scheme 11, Compound 26 (0.31 g, 0.7 mmol), triethylamine (0.10 mL, 0.7 mmol), formaldehyde (0.15 mL), and palladium catalyst (0.15 g) were reacted to synthesize Compound 31 in the form of white powder; ¹H NMR (DMSO-d₆, 400 MHz) 8.22 (t, J = 539 Hz, C(O)NH), 7.63-7.79 (m, ArH), 7.17-7.22 (m, ArH), 6.94-6.99 (m, ArH), 5.21 (s, OCH₂), 4.19-4.24 (m, NHCH₂), 2.70-2.76 (NCH), 2.18 (s, N(CH₃)₂), 1.65-1.73 (m, CH₂CH₂CH₃), 1.20-1.35 (m, CH₂CH₂CH₃), 0.86 (t, J = 7.4 Hz, CH₂CH₂CH₃).

### Preparation example 30:

### (R)/(S)-tert-butyl-2-((4-(3-fluorobenzyl)oxy)benzyl)carbamoyl)pyrrolidine-1-carboxylate (Compound 34)

Using the above Scheme 11, Compound 33 (2.00 g, 9.3 mmol), 3-fluorobenzyl bromide (2.63 g, 11.2 mmol), and K₂CO₃ (5.14 g, 37.2 mmol) were reacted to synthesize Compound 34 in the form of white powder (3.91 g, 98%): Rε = 0.27 (EtOAc 1 : n-hexane 5); ¹H NMR (DMSO-d₆, 400 MHz) 8.27 (t, J = 5.8 Hz, C(O)NH), 7.35-7.50 (m, ArH), 7.20-7.30 (m, ArH), 7.10-7.20 (m, ArH), 6.93 (d, J = 7.8 Hz, Boc-NH), 5.12 (s, OCH₂), 4.00-4.30 (m,NCH, NHCH₂), 3.02-3.45 (NCHCH₂CH₂CH₂), 1.70-2.15 (m,NCHCH₂CH₂CH₂), 1.30 (s, Boc).

### Preparation example 31:

### (R)/(S)-tert-butyl-2-((4-((4-fluorobenzyl)oxy)benzyl)carbamoyl)pyrrolidine-1-carboxylate (Compound 35)

Using the above Scheme 11, Compound 33, 4-fluorobenzyl bromide, and K₂CO₃ were reacted to synthesize Compound 35 in the form of white powder; ¹H NMR (DMSO-d₆, 400 MHz) 8.27 (t, J = 5.8 Hz, C(O)NH), 7.35-7.45 (m, ArH), 7.15-7.25 (m, ArH), 7.05-7.10 (m, ArH), 6.85-6.95 (m, ArH), 5.01 (s, OCH₂), 4.20-4.60 (m, NCH, NHCH₂), 2.10-2.50 (NCHCH₂CH₂CH₂), 1.80-2.00 (m,NCHCH₂CH₂CH₂), 1.25 (s, Boc).

### Preparation example 32:

### (R)/(S)-tert-butyl-2-((4-((4-(trifluoromethyl)benzyl)oxy)benzyl)carbamoyl)pyrrolidine-1-carboxylate (Compound 36)

Using the above Scheme 11, Compound 33, 4-(trifluoromethyl)benzyl bromide, and K₂CO₃ were reacted to synthesize Compound 36 in the form of white powder; ¹H NMR (DMSO-d₆, 400 MHz) 8.25 (t, J = 5.7 Hz, C(O)NH), 7.70-7.60 (m, ArH), 7.60-7.50 (m, ArH), 7.15-7.25 (m, ArH), 6.85-6.95 (m, ArH), 5.11 (s, OCH₂), 4.20-4.60 (m,NCH, NHCH₂), 2.10-2.40 (NCHCH₂CH₂CH₂), 1.80-1.95 (m,NCHCH₂CH₂CH₂), 1.25 (s, Boc).

### Preparation example 33:

### (R)/(S)-tert-butyl-2-((4-(4-(chlorobenzyl)oxy)benzyl)carbamoyl)pyrrolidine-1-carboxylate (Compound 37)

Using the above Scheme 11, Compound 33, 4-chlorobenzyl bromide, and K₂CO₃ were reacted to synthesize Compound 37 in the form of white powder; ¹H NMR (DMSO-d₆, 400 MHz) 8.28 (t, J = 5.9 Hz, C(O)NH), 7.70-7.60 (m, ArH), 7.60-7.50 (m, ArH), 7.15-7.25 (m, ArH), 6.85-6.95 (m, ArH), 5.11 (s, OCH₂), 4.20-4.60 (m,NCH, NHCH₂), 2.10-2.40 (NCHCH₂CH₂CH₂), 1.80-1.95 (m,NCHCH₂CH₂CH₂), 1.27 (s, Boc).

### Preparation example 34:

### (R)/(S)-N-(4-((3-fluorobenzyl)oxy)benzyl)pyrrolidine-2-carboxamide hydrochloride (Compound 38)

Using the above Scheme 9, Compound 34 (1.00 g, 2.3 mmol) and 4.0 M HCl in dioxane (7.32 mL, 233.0 mmol) were reacted to synthesize Compound 38 in the form of white powder (0.77 g, 100%): Rε = 0.00 (EtOAc 9 : Acetone 1); ¹H NMR (DMSO-d₆, 400 MHz) 8.99 (t, J = 5.5 Hz, C(O)NH), 8.55 (broad, NH₂⁺Cl⁻), 7.39-7.47 (m, ArH), 7.23-7.30 (m, ArH), 7.18-7.23 (m, ArH), 7.10-7.18 (m, ArH), 6.92-7.01 (m, ArH), 5.13 (s, OCH₂), 4.23-4.30 (m, NHCH₂), 4.11-4.19 (m, NHCH), 3.14-3.27 (m, NHCHCH₂CH₂CH₂), 2.24-2.34 (m, NHCHCH₂CHH'CH₂), 1.77-1.93 (m, NHCHCH₂CHH'CH₂).

### Preparation example 35:

### (R)/(S)-N-(4-((4-fluorobenzyl)oxy)benzyl)pyrrolidine-2-carboxamide hydrochloride (Compound 39)

Using the above Scheme 9, Compound 35 (1.00 g, 2.3 mmol) and 4.0 M HCl in dioxane (4.05 mL, 117.0 mmol) were reacted to synthesize Compound 39 in the form of white powder (0.84 g, 99%): Rε = 0.00 (EtOAc 9 : Acetone 1); ¹H NMR (DMSO-d₆, 400 MHz) 9.04 (t, J = 5.7 Hz, C(O)NH), 8.55 (broad, NH₂⁺Cl⁻), 7.46-7.53 (m, ArH), 7.18-7.26 (m, ArH), 6.95-7.01 (m, ArH), 5.08 (s, OCH₂), 4.25-4.31 (m, NHCH₂), 4.12-4.21 (m, NHCH), 3.13-3.29 (m, NHCHCH₂CHHCH₂), 2.25-2.35 (m, NHCHCH₂CHHCH₂), 1.77-1.94 (m, NHCHCH₂CHHCH₂).

### Preparation example 36:

### (R)/(S)-N-(4-((4-(trifluoromethyl)benzyl)oxy)benzyl)pyrrolidine-2-carboxamide hydrochloride (Compound 40)

Using the above Scheme 9, Compound 36 (1.00 g, 2.09 mmol) and 4.0 M HCl in dioxane (3.63 mL, 104.0 mmol) were reacted to synthesize Compound 40 in the form of white powder (0.85 g, 98%): Rε = 0.00 (EtOAc 9 : Acetone 1); ¹H NMR (DMSO-d₆, 400 MHz) 8.98 (t, J = 5.8 Hz, C(O)NH), 8.56 (broad, NH₂⁺Cl⁻), 7.63-7.79 (m, ArH), 7.19-7.25 (m, ArH), 6.97-7.03 (m, ArH), 5.23 (s, OCH₂), 4.26-4.30 (m, NHCH₂), 4.12-4.18 (m, NHCH), 3.13-3.27 (m, NHCHCH₂CH₂CH₂), 2.23-2.35 (m, NHCHCH₂CHH'CH₂), 1.77-1.93 (m, NHCHCH₂CHH'CH₂).

### Preparation example 37:

### (R)/(S)-N-(4-((4-chlorobenzyl)oxy)benzyl)pyrrolidine-2-carboxamide hydrochloride (Compound 41)

Using the above Scheme 9, Compound 37 (1.00 g, 2.09 mmol) and 4.0 M HCl in dioxane (3.63 mL, 104.0 mmol) were reacted to synthesize Compound 41 in the form of white powder (0.85 g, 98%): Rε = 0.00 (EtOAc 9 : Acetone 1); ¹H NMR (DMSO-d₆, 400 MHz) 8.97 (t, J = 5.4 Hz, C(O)NH), 8.57 (broad, NH₂⁺Cl⁻), 7.40-7.48 (m, ArH), 7.20-7.29 (m, ArH), 7.18-7.23 (m, ArH), 7.08-7.18 (m, ArH), 6.95-7.05 (m, ArH), 5.11 (s, OCH₂), 4.20-4.31 (m, NHCH₂), 4.09-4.19 (m, NHCH), 3.14-3.27 (m, NHCHCH₂CH₂CH₂), 2.25-2.36 (m, NHCHCH₂CHH'CH₂), 1.70-1.83 (m, NHCHCH₂CHH'CH₂).

### Preparation example 38:

### (R)/(S)-1-methyl-N-(4-((3-fluorobenzyl)oxy)benzyl)pyrrolidine-2-carboxamide (Compound 42)

Using the above Scheme 11, Compound 38 (0.31 g, 0.7 mmol), formaldehyde (0.15 mL), and palladium catalyst (0.15 g) were reacted to synthesize Compound 42 in the form of white powder; ¹H NMR (DMSO-d₆, 400 MHz) 7.57 (t, J = 5.2 Hz, C(O)NH), 7.36-7.43 (m, ArH), 7.17-7.28 (m, ArH), 7.01-7.08 (m, ArH), 6.95-7.00 (m, ArH), 5.05 (s, OCH₂), 4.33-4.37 (m, NHCH₂), 3.10-3.17 (m, NCHCHHCH₂CH₂), 2.85-2.91 (m, NCHCHHCH₂CH₂), 2.34 (s, NCH₃), 2.17-2.32 (m, NCHCH₂CH₂CH₂), 1.82-1.92 (m, NCHCH₂CH₂CH₂), 1.67-1.82 (m, NCHCH₂CH₂CH₂).

### Preparation example 39:

### (R)/(S)-1-methyl-N-(4-((4-(trifluoromethyl)benzyl)oxy)benzyl)pyrrolidine-2-carboxamide (Compound 43)

Using the above Scheme 11, Compound 40 (0.31 g, 0.7 mmol), triethylamine (0.10 mL, 0.7 mmol), formaldehyde (0.15 mL), and palladium catalyst (0.15 g) were reacted to synthesize Compound 43 in the form of white powder; ¹H NMR (DMSO-d₆, 400 MHz) 7.62-7.67 (m, ArH), 7.52-7.62 (t, J = 5.4 Hz, C(O)NH, m, ArH), 7.18-7.23 (m, ArH), 6.90-6.96 (m, ArH), 5.12 (s, OCH₂), 4.33-4.45 (m, NHCH₂), 3.00-3.10 (m, NCHCH₂CH₂CH₂), 2.90-2.98 (m, NCHCHHCH₂CH₂), 2.37 (s, NCH₃), 2.30-2.40 (m, NCHCH₂CH₂CH₂), 2.18-2.30 (m, NCHCHHCH₂CH₂).

### Preparation example 40:

### (R)/(S)-1-methyl-N-(4-((4-chlorobenzyl)oxy)benzyl)pyrrolidine-2-carboxamide (Compound 44)

Using the above Scheme 11, Compound 41 (0.31 g, 0.7 mmol), formaldehyde (0.15 mL), and palladium catalyst (0.15 g) were reacted to synthesize Compound 44 in the form of white powder; ¹H NMR (DMSO-d₆, 400 MHz) 7.75 (t, J = 5.4 Hz, C(O)NH), 7.60-7.67 (m, ArH), 7.52-7.62 (m, ArH), 7.18-7.23 (m, ArH), 6.90-7.00 (m, ArH), 5.11 (s, OCH₂), 4.34-4.45 (m, NHCH₂), 3.00-3.09 (m, NCHCH₂CH₂CH₂), 2.90-2.99 (m, NCHCHHCH₂CH₂), 2.35 (s, NCH₃), 2.28-2.40 (m, NCHCH₂CH₂CH₂), 2.18-2.30 (m, NCHCHHCH₂CH₂).

### Preparation example 41:

### (R)/(S)-tert-butyl-3-((4((3-fluorobenzyl)oxy)benzyl)carbamoyl)pyrolidine-1-carboxylate (Compound 46)

Using the above Scheme 7, Compound 45 (1.00 g, 4.6 mmol), NMM (0.77 mL, 7.0 mmol), IBCF (0.76 mL, 5.8 mmol), and 4-[(3-fluorophenyl)methoxy]-benzenemethaneamine hydrochloride (1.47 g, 5.5 mmol) were reacted to synthesize Compound 46 in the form of white powder (1.97 g, 99%): Rε = 0.30 (EtOAc 1 : n-hexane 1); ¹H NMR (DMSO-d₆, 400 MHz) 7.57 (t, J = 5.2 Hz, C(O)NH), 7.32-7.39 (m, ArH), 7.11-7.24 (m, ArH), 6.89-6.94 (m, ArH), 5.07 (s, OCH₂), 4.33-4.37 (m, NHCH₂), 3.21-3.68 (m, NCH₂CHCH₂CH₂), 2.85-2.98 (m, NCH₂CHCH₂CH₂), 2.00-2.12 (m, NCH₂CHCH₂CH₂), 1.45 (s, Boc).

### Preparation example 42:

### (R)/(S)-tert-butyl-3-((4((4-(trifluoromethyl)benzyl)oxy)benzyl)carbamoyl)pyrrolidine-1-carboxylate (Compound 47)

Using the above Scheme 7, Compound 45 (1.00 g, 4.6 mmol), NMM (0.77 mL, 7.0 mmol), IBCF (0.76 mL, 5.8 mmol), and 4-[(4-(trifluoromethyl)phenyl)methoxy]-benzenemethaneamine hydrochloride (1.75 g, 5.5 mmol) were reacted to synthesize Compound 47 in the form of white powder (2.20 g, 99%): Rε = 0.33 (EtOAc 1 : n-hexane 1); ¹H NMR (DMSO-d₆, 400 MHz) 7.78 (t, J = 5.3 Hz, C(O)NH), 7.63-7.67 (m, ArH), 7.52-7.57 (m, ArH), 7.18-.23 (m, ArH), 6.90-6.95 (m, ArH), 5.12 (s, OCH₂), 4.37-4.42 (m, NHCH₂), 3.53-3.67 (m, NCH₂CHCH₂CH₂), 3.47-3.53 (m, NCH₂CHCH₂CH₂), 2.78-2.89 (m, NCH₂CHCH₂CH₂), 2.06-2.13 (m, NCH₂CHCH₂CH₂), 1.45 (s, Boc).

### Preparation example 43:

### (R)/(S)-tert-butyl-3-((4-(4-chlorobenzyl)oxy)benzyl)carbamoyl)pyrrolidine-1-carboxylate (Compound 48)

Using the above Scheme 7, Compound 45 (1.00 g, 4.6 mmol), NMM (0.77 mL, 7.0 mmol), IBCF (0.76 mL, 5.8 mmol), and 4-[(4-(trifluoromethyl)phenyl)methoxy]-benzenemethaneamine hydrochloride (1.75 g, 5.5 mmol) were reacted to synthesize Compound 48 in the form of white powder (2.20 g, 99%): Rε = 0.33 (EtOAc 1 : n-hexane 1); ¹H NMR (DMSO-d₆, 400 MHz) 7.80 (t, J = 5.4 Hz, C(O)NH), 7.63-7.67 (m, ArH), 7.52-7.57 (m, ArH), 7.18-7.23 (m, ArH), 6.90-6.95 (m, ArH), 5.12 (s, OCH₂), 4.37-4.42 (m, NHCH₂), 3.53-3.67 (m, NCH₂CHCH₂CH₂), 3.47-3.53 (m, NCH₂CHCH₂CH₂), 2.78-2.89 (m, NCH₂CHCH₂CH₂), 2.06-2.13 (m, NCH₂CHCH₂CH₂), 1.45 (s, Boc).

### Preparation example 44:

### (R)/(S)-2-((4-((3-fluorobenzyl)oxy)benzyl)carbamoyl)pyrrolidi-1-nium chloride (Compound 49)

Using the above Scheme 9, Compound 46 (1.00 g, 2.3 mmol) and 4.0 M HCl in dioxane (4.05 mL, 116.7 mmol) were reacted to synthesize Compound 49 in the form of white powder (0.84 g, 99%): Rε = 0.00 (EtOAc 9 : Acetone 1); ¹H NMR (DMSO-d₆, 400 MHz) 9.13 (broad, NH₂⁺Cl⁻), 8.65 (t, J = 5.6 Hz, C(O)NH), 7.40-7.48 (m, ArH), 7.23-7.30 (m, ArH), 7.12-7.21 (m, ArH), 6.94-7.00 (m, ArH), 5.12 (s, OCH₂), 4.15-4.27 (m, NHCH₂), 3.04-3.32 (m, NHCH₂CHCH₂CH₂), 1.88-2.20 (m, NHCH₂CHCH₂CH₂).

### Preparation example 45:

### (R)/(S)-2-((4-((4-(trifluoromethyl)benzyl)oxy)benzyl)carbamoyl)pyrrolidi-1-nium chloride (Compound 50)

Using the above Scheme 9, Compound 47 (1.00 g, 2.09 mmol) and 4.0 M HCl in dioxane (3.63 mL, 104.5 mmol) were reacted to synthesize Compound 50 in the form of white powder (0.86 g, 99%): Rε = 0.00 (EtOAc 9 : Acetone 1); ¹H NMR (DMSO-d₆, 400 MHz) 9.13 (broad, NH₂⁺Cl⁻), 8.65 (t, J = 5.6 Hz, C(O)NH), 7.60-7.71 (m, ArH), 7.20-7.27 (m, ArH), 6.95-7.01 (m, ArH), 5.20 (s, OCH₂), 4.28-4.35 (m, NHCH₂), 3.36-3.56 (m, NHCH₂CHCH₂CH₂), 2.27-2.39 (m, NHCH₂CHCH₂CH₂), 2.10-2.20 (m, NHCH₂CHCH₂CH₂).

### Preparation example 46:

### (R)/(S)-2-((4-(4-chlorobenzyl)oxy)benzyl)carbamoyl)pyrrolidi-1-nium chloride (Compound 51)

Using the above Scheme 9, Compound 48 (1.00 g, 2.09 mmol) and 4.0 M HCl in dioxane (3.63 mL, 104.5 mmol) were reacted to synthesize Compound 51 in the form of white powder (0.86 g, 99%): Rε = 0.00 (EtOAc 9 : Acetone 1); ¹H NMR (DMSO-d₆, 400 MHz) 9.12 (broad, NH₂⁺Cl⁻), 8.63 (t, J = 5.6 Hz, C(O)NH), 7.60-7.70 (m, ArH), 7.20-7.29 (m, ArH), 6.92-7.01 (m, ArH), 5.18 (s, OCH₂), 4.27-4.35 (m, NHCH₂), 3.38-3.56 (m, NHCH₂CHCH₂CH₂), 2.25-2.40 (m, NHCH₂CHCH₂CH₂), 2.10-2.20 (m, NHCH₂CHCH₂CH₂).

### Preparation example 47:

### (R)/(S)-1-methyl-N-(4-((3-fluorobenzyl)oxy)benzyl)pyrrolidine-3-carboxamide (Compound 52)

Using the above Scheme 11, Compound 49 (0.14 g, 0.4 mmol), formaldehyde (0.03 mL), and palladium catalyst (0.07 g) were reacted to synthesize Compound 52 in the form of white powder; ¹H NMR (DMSO-d₆, 400 MHz) 7.57 (t, J = 5.2 Hz, C(O)NH), 7.36-7.43 (m, ArH), 7.16-7.28 (m, ArH), 7.01-7.08 (m, ArH), 6.94-6.99 (m, ArH), 5.11 (s, OCH₂), 4.29-4.31 (m, NHCH₂), 2.93-3.05 (m, NHCH₂CHCH₂CH₂), 2.78-2.85 (m, NHCH₂CHCH₂CH₂), 2.50-2.64 (m, NHCH₂CHCH₂CH₂), 2.39 (s, NCH₃), 2.02-2.15 (m, m, NHCH₂CHCH₂CH₂).

### Preparation example 48:

### (R)/(S)-1-methyl-N-(4-((4-(trifluoromethyl)benzyl)oxy)benzyl)pyrrolidine-3-carboxamide (Compound 53)

Using the above Scheme 11, Compound 50 (0.14 g, 0.4 mmol), formaldehyde (0.03 mL), and palladium catalyst (0.07 g) were reacted to synthesize Compound 53 in the form of white powder; ¹H NMR (DMSO-d₆, 400 MHz) 7.62-7.69 (m, ArH), 7.52-7.57 (m, ArH), 7.17-7.23 (m, ArH), 7.02 (t, J = 5.4 Hz, C(O)NH), 6.89-6.95 (m, ArH), 5.12 (s, OCH₂), 4.30-4.45 (m, NHCH₂), 2.75-2.95 (m, NCH₂CH₂CHCH₂), 2.27-2.50 (m, NCH₂CH₂CHCH₂), 2.35 (s, NCH₃), 1.95-2.25 (m, NCH₂CHCH₂CH₂).

### Preparation example 49:

### (R)/(S)-1-methyl-N-(4-((4-chlorobenzyl)oxy)benzyl)pyrrolidine-3-carboxamide (Compound 54)

Using the above Scheme 11, Compound 51 (0.14 g, 0.4 mmol), formaldehyde (0.03 mL), and palladium catalyst (0.07 g) were reacted to synthesize Compound 54 in the form of white powder; ¹H NMR (DMSO-d₆, 400 MHz) 7.60-7.69 (m, ArH), 7.50-7.57 (m, ArH), 7.17-7.25 (m, ArH), 7.05 (t, J = 5.4 Hz, C(O)NH), 6.89-6.95 (m, ArH), 5.10 (s, OCH₂), 4.32-4.43 (m, NHCH₂), 2.75-2.95 (m, NCH₂CH₂CHCH₂), 2.25-2.51 (m, NCH₂CH₂CHCH₂), 2.34 (s, NCH₃), 1.94-2.27 (m, NCH₂CHCH₂CH₂).

### Preparation example 50:

### (R)/(S)-tert-butyl(1-((4-((3,4-chlorobenzyl)oxy)benzyl)amino)-1-oxobuta-2-nyl)carbamate (Compound 55)

Using the above Scheme 8, Compound 5 (0.20 g, 0.7 mmol), 3,4-chlorobenzyl bromide (0.09 mL, 0.7 mmol), and K₂CO₃ (0.36 g, 2.6 mmol) were reacted to synthesize Compound 55 in the form of white powder (0.28 g, 100%): R_{f} = 0.50 (EtOAc 1 : n-hexane 1); ¹H NMR (DMSO-d₆, 400 MHz) 8.21 (t, J = 5.9 Hz, C(O)NH), 7.42-7.52 (m, ArH), 7.13-7.20 (m, ArH), 6.90-6.95 (m, ArH), 6.80 (d, J = 8.0 Hz, Boc-NH), 5.08 (s, OCH₂), 4.13-4.27 (m, NHCH₂), 3.79-3.87 (m, NHCHCH₂), 1.45-1.68 (m, CH₂CH₃), 1.37 (s, Boc), 0.82 (t, J = 7.3 Hz, CH₂CH₃).

### Preparation example 51:

### (R)/(S)-tert-butyl(1-oxo-1-((4-((4-(trifluoromethyl)benzyl)oxy)benzyl)amino)penta-2-nyl)carbamate (Compound 56)

Using the above Scheme 8, Compound 6 (1.00 g, 3.2 mmol), 3,4-dichlorobenzyl bromide (0.57 mL, 3.9 mmol), and K₂CO₃ (1.79 g, 13.0 mmol) were reacted to synthesize Compound 56 in the form of white powder (0.75 g, 50%): Rε = 0.24 (EtOAc 1 : n-hexane 2); ¹H NMR (DMSO-d₆, 400 MHz) 8.22 (t, J = 5.9 Hz, C(O)NH), 7.60-7.75 (m, ArH), 7.38-7.48 (m, ArH), 7.10-7.25 (m, ArH), 6.85-6.98 (m, ArH), 6.82 (d, J = 7.8 Hz, Boc-NH), 5.12 (s, OCH₂), 4.10-4.30 (m, NHCH₂), 3.78-3.90 (m, NHCHCH₂), 1.41-1.70 (m, CH₂CH₃), 1.38 (s, Boc), 0.87 (t, J = 7.8 Hz, CH₂CH₃).

### Preparation example 52:

### (R)/(S)-1-((4-(3,4-dichlorobenzyl)oxy)benzyl)amino)-1-oxobutane-2-aminium chloride (Compound 57)

Using the above Scheme 9, Compound 55 (0.29 g, 0.6 mmol) and 4.0 M HCl in dioxane (1.05 mL, 30.1 mmol) were reacted to synthesize Compound 57 in the form of white powder (0.24 g, 95%): Rε = 0.30 (CH₂Cl₂ 9 : MeOH 1); ¹H NMR (DMSO-d₆, 400 MHz) 8.85 (t, J = 5.6 Hz, C(O)NH), 8.10 (broad, NH₃⁺Cl⁻), 7.63-7.75 (m, ArH), 7.40-7.50 (m, ArH), 7.18-7.30 (m, ArH), 6.93-7.03 (m, ArH), 5.13 (s, OCH₂), 4.20-4.38 (m, NHCH₂), 3.65-3.78 (m, NH₃CHCH₂), 1.70-1.80 (m, CH₂CH₃), 0.87 (t, J = 7.4 Hz, CH₂CH₃).

### Preparation example 53:

### (R)/(S)-1-((4-((3,4-dichlorobenzyl)oxy)benzyl)amino)-1-oxopentane-2-aminium chloride (Compound 58)

Using the above Scheme 9, Compound 56 (0.65 g, 1.4 mmol) and 4.0 M HCl in dioxane (2.34 mL, 67.5 mmol) were reacted to synthesize Compound 58 in the form of white powder (0.39 g, 70%): Rε = 0.00 (EtOAc 9 : Acetone 1); ¹H NMR (DMSO-d₆, 400 MHz) 8.99 (t, J = 7.3 Hz, C(O)NH), 8.26 (broad, NH₃⁺Cl⁻), 7.60-7.78 (m, ArH), 7.38-7.48 (m, ArH), 7.10-7.40 (m, ArH), 6.90-7.10 (m, ArH), 5.12 (s, OCH₂), 4.18-4.38 (m, NHCH₂), 3.70-3.83 (m, NH₃CHCH₂), 1.60-1.80 (m, CH₂CH₂CH₃), 1.20-1.40 (m, CH₂CH₂CH₃), 0.86 (t, J = 9.6 Hz, CH₂CH₂CH₃).

### Preparation example 54:

### (R)/(S)-2-(dimethylamino)-N-(4-((4-trifluoromethyl)benzyl)oxy)benzyl)butanamide (Compound 59)

Using the above Scheme 11, Compound 22 (0.10 g, 0.2 mmol), triethylamine (0.20 mL, 1.4 mmol), formaldehyde (0.03 mL), and palladium catalyst (0.10 g) were reacted to synthesize Compound 59 in the form of white powder; ¹H NMR (DMSO-d₆, 400 MHz) 8.23 (t, J = 5.9 Hz, C(O)NH), 7.73-7.79 (m, ArH), 7.62-7.70 (m, ArH), 7.16-7.23 (m, ArH), 6.93-6.99 (m, ArH), 5.21 (s, OCH₂), 4.17-4.24 (m, NHCH₂), 2.70-2.76 (NCH), 2.18 (s, N(CH₃)₂), 1.48-1.63 (m, CH₂CH₃), 0.78 (t, J = 7.4 Hz, CH₂CH₃).

### Preparation example 55:

### (R)/(S)-N-(4-((3,4-dichlorobenzyl)oxy)benzyl)-2-(dimethylamino)butanamide (Compound 60)

Using the above Scheme 11, Compound 57 (0.20 g, 0.5 mmol), triethylamine (0.40 mL, 2.9 mmol), formaldehyde (0.11 mL), and palladium catalyst (0.04 g) were reacted to synthesize Compound 60 in the form of white powder; ¹H NMR (DMSO-d₆, 400 MHz) 8.23 (t, J = 5.8 Hz, C(O)NH), 7.63-7.72 (m, ArH), 7.30-7.47 (m, ArH), 7.15-7.22 (m, ArH), 6.93-6.99 (m, ArH), 5.11 (s, OCH₂), 4.10-4.30 (m, NHCH₂), 2.80-2.90 (NCH), 2.19 (s, N(CH₃)₂), 1.40-1.63 (m, CH₂CH₂CH₃), 1.25-1.30 (m, CH₂CH₂CH₃), 0.86 (t, J = 7.4 Hz, CH₂CH₂CH₃).

### Preparation example 56:

### (R)/(S)-N-(4-((3,4-dichlorobenzyl)oxy)benzyl)-2-(dimethylamino)butanamide (Compound 61)

Using the above Scheme 11, Compound 58 (0.31 g, 0.7 mmol), triethylamine (0.10 mL, 0.7 mmol), formaldehyde (0.15 mL), and palladium catalyst (0.15 g) were reacted to synthesize Compound 61 in the form of white powder; ¹H NMR (DMSO-d₆, 400 MHz) 8.23 (t, J = 5.7 Hz, C(O)NH), 7.60-7.72 (m, ArH), 7.30-7.49 (m, ArH), 7.10-7.25 (m, ArH), 6.92-7.00 (m, ArH), 5.15 (s, OCH₂), 4.10-4.30 (m, NHCH₂), 2.79-2.89 (NCH), 2.18 (s, N(CH₃)₂), 1.40-1.60 (m, CH₂CH₂CH₃), 1.23-1.31 (m, CH₂CH₂CH₃), 0.87 (t, J = 7.4 Hz, CH₂CH₂CH₃).

### Preparation example 57:

### (R)/(S)-2-(ethylamino)-N-(4-((4-trifluoromethyl)benzyl)oxy)benzyl)butanamide (Compound 62)

Using the above Scheme 12, Compound 22 (0.10 g, 0.3 mmol), triethylamine (0.21 mL, 1.5 mmol), and iodoethane (0.10 mL) were reacted to synthesize Compound 62 in the form of white powder; ¹H NMR (DMSO-d₆, 400 MHz) 7.61-7.67 (m, ArH), 7.51-7.60 (m, ArH), 7.17-7.25 (m, ArH), 6.89-6.95 (m, ArH), 5.12 (s, OCH₂), 4.34-4.45 (m, NHCH₂), 3.03-3.09 (NCH), 2.52-2.67 (m, CH₂CH₂CH₃), 1.75-1.87 (m, CHHCH₃), 1.55-1.68 (m, CHHCH₃, CH₂CH₂CH₃), 1.06 (t, J = 7.1 Hz, CH₂CH₃), 0.95 (t, J = 7.5 Hz, CH₂CH₂CH₃).

### Preparation example 58:

### (R)/(S)-N-(4-((3,4-dichlorobenzyl)oxy)benzyl-2-(ethylamino)butanamide (Compound 63)

Using the above Scheme 12, Compound 57 (0.10 g, 0.7 mmol), triethylamine (0.21 mL, 1.5 mmol), and iodoethane (0.10 mL) were reacted to synthesize Compound 63 in the form of white powder; ¹H NMR (DMSO-d₆, 400 MHz) 7.61-7.70 (m, ArH), 7.50-7.60 (m, ArH), 7.25-7.38 (m, ArH), 6.99-7.15 (m, ArH), 5.10 (s, OCH₂), 4.30-4.45 (m, NHCH₂), 3.10-3.25 (NCH), 2.48-2.67 (m, CH₂CH₂CH₃), 1.75-1.88 (m, CHHCH₃), 1.53-1.69 (m, CHHCH₃, CH₂CH₂CH₃), 1.06 (t, J = 7.1 Hz, CH₂CH₃), 0.93 (t, J = 7.4 Hz, CH₂CH₂CH₃).

### Preparation example 59:

### (R)/(S)-2-(ethylamino)-N-(4-((4-(trifluoromethyl)benzyl)oxy)benzyl)pentanamide (Compound 64)

Using the above Scheme 12, Compound 26 (0.13 g, 0.3 mmol), triethylamine (0.26 mL, 1.9 mmol), and iodoethane (0.02 mL) were reacted to synthesize Compound 64 in the form of white powder; ¹H NMR (DMSO-d₆, 400 MHz) 7.63-7.80 (m, ArH), 7.18-7.30 (m, ArH), 6.90-7.00 (m, ArH), 5.20 (s, OCH₂), 4.19-4.38 (m, NHCH₂), 3.35-3.42 (m, CHCH₂CH₂CH₃), 1.63-1.73 (m, NH), 1.33-1.48 (m, CHHCH₂CH₃CH₂CH₃), 1.15-1.19 (m, CHHCH₂CH₃), 1.10-1.15 (m, CH₂CH₃), 0.90 (t, J = 7.1 Hz, CH₂CH₂CH₃).

### Preparation example 60:

### (R)/(S)-N-(4-((3,4-dichlorobenzyl)oxy)benzyl-2-(ethylamino)pentanamide (Compound 65)

Using the above Scheme 12, Compound 58 (0.20 g, 0.5 mmol), triethylamine (0.40 mL, 2.9 mmol), and iodotetaine (0.04 mL, 0.4 mmol) were reacted to synthesize Compound 65 in the form of white powder; ¹H NMR (DMSO-d₆, 400 MHz) 8.26 (t, J = 5.9 Hz, C(O)NH), 7.68-7.72 (m, ArH), 7.62-7.68 (m, ArH), 67.40-7.46 (m, ArH), 7.14-7.20 (m, ArH), 6.91-6.99 (m, ArH), 5.11 (s, OCH₂), 4.18-4.25 (m, NHCH₂), 2.92-2.98 (NCH), 2.31-2.48 (m, CH₂CH₂CH₃), 1.35-1.50 (m, CH₂CH₃), 1.20-1.35 (m, CH₂CH₂CH₃), 0.96 (t, J =7.1 Hz, CH₂CH₃), 0.84 (t, J = 7.2 Hz, CH₂CH₂CH₃).

### Preparation example 61:

### (R)/(S)-2-(diethylamino)-N-(4-((4-(trifluoromethyl)benzyl)oxy)benzyl)butanamide (Compound 66)

Using the above Scheme 12, Compound 22 (0.10 g, 0.3 mmol), triethylamine (0.21 mL, 1.5 mmol), and iodoethane (0.10 mL) were reacted to synthesize Compound 66 in the form of white powder; ¹H NMR (DMSO-d₆, 400 MHz) 8.20 (t, J = 5.4 Hz, NHCH₂), 7.57-7.70 (m, ArH), 7.10-7.22(m, ArH), 6.88-7.02 (m, ArH), 5.25 (s, OCH₂), 4.08-4.23 (m, NHCH₂), 3.05-3.13 (m, CHCH₂CH₃), 2.52-2.63 (m, CH₂CH₃), 2.35-2.45 (m, CH₂CH₃), 1.42-1.67 (m, CH₂CH₃), 0.93 (t, J = 7.0 Hz, CH₂CH₃, CH₂CH₃), 0.84 (t, J = 7.4 Hz, CH₂CH₃).

### Preparation example 62:

### (R)/(S)-N-(4-((3,4-dichlorobenzyl)oxy)benzyl-2-(diethylamino)butanamide (Compound 67)

Using the above Scheme 12, Compound 57 (0.10 g, 0.7 mmol), triethylamine (0.21 mL, 1.5 mmol), and iodoethane (0.10 mL) were reacted to synthesize Compound 67 in the form of white powder; ¹H NMR (DMSO-d₆, 400 MHz) 8.16 (t, J = 5.3 Hz, NHCH₂), 7.57-7.72 (m, ArH), 7.10-7.25(m, ArH), 6.93-7.02 (m, ArH), 5.21 (s, OCH₂), 4.10-4.26 (m, NHCH₂), 3.10-3.18 (m, CHCH₂CH₃), 2.50-2.63 (m, CH₂CH₃), 2.35-2.48 (m, CH₂CH₃), 1.35-1.55 (m, CH₂CH₃), 0.92 (t, J = 7.0 Hz, CH₂CH₃, CH₂CH₃), 0.84 (t, J = 7.4 Hz, CH₂CH₃).

### Preparation example 63:

### (R)/(S)-2-(diethylamino)-N-(4-((4-(trifluoromethyl)benzyl)oxy)benzyl)pentanamide Compound (68)

Using the above Scheme 12, Compound 26 (0.13 g, 0.3 mmol), triethylamine (0.26 mL, 1.9 mmol), and iodoethane (0.02 mL) were reacted to synthesize 68 in the form of white powder; ¹H NMR (DMSO-d₆, 400 MHz) 8.16 (t, J = 5.4 Hz, NHCH₂), 7.60-7.80 (m, ArH), 7.15-7.25(m, ArH), 6.90-7.00 (m, ArH), 5.21 (s, OCH₂), 4.12-4.26 (m, NHCH₂), 3.09-3.15 (m, CHCH₂CH₂CH₃), 2.52-2.63 (m, CH₂CH₃), 2.38-2.48 (m, CH₂CH₃), 1.40-1.60 (m, CH₂CH₂CH₃), 1.20-1.31 (m, CH₂CH₂CH₃), 0.93 (t, J = 7.1 Hz, CH₂CH₃, CH₂CH₃), 0.86 (t, J = 7.3 Hz, CH₂CH₃).

### Preparation example 64:

### (R)/(S)-N-(4-((3,4-dichlorobenzyl)oxy)benzyl-2-(diethylamino)pentanamide (Compound 69)

Using the above Scheme 12, Compound 58 (0.20 g, 0.5 mmol), triethylamine (0.40 mL, 2.9 mmol), and iodotetaine (0.04 mL, 0.4 mmol) were reacted to synthesize Compound 69 in the form of white powder; ¹H NMR (DMSO-d₆, 400 MHz) 8.20 (t, J = 5.4 Hz, NHCH₂), 7.60-7.80 (m, ArH), 7.13-7.22(m, ArH), 6.90-7.02 (m, ArH), 5.20 (s, OCH₂), 4.12-4.25 (m, NHCH₂), 3.10-3.15 (m, CHCH₂CH₂CH₃), 2.50-2.63 (m, CH₂CH₃), 2.37-2.49 (m, CH₂CH₃), 1.40-1.60 (m, CH₂CH₂CH₃), 1.20-1.33 (m, CH₂CH₂CH₃), 0.92 (t, J = 7.1 Hz, CH₂CH₃, CH₂CH₃), 0.86 (t, J = 7.4 Hz, CH₂CH₃).

### Experimental Examples

### Example 1: In vitro anti-inflammatory effect

### Example 1-1: Confirmation of Anti-Inflammatory Effect Using Interleukin-6 (IL-6)

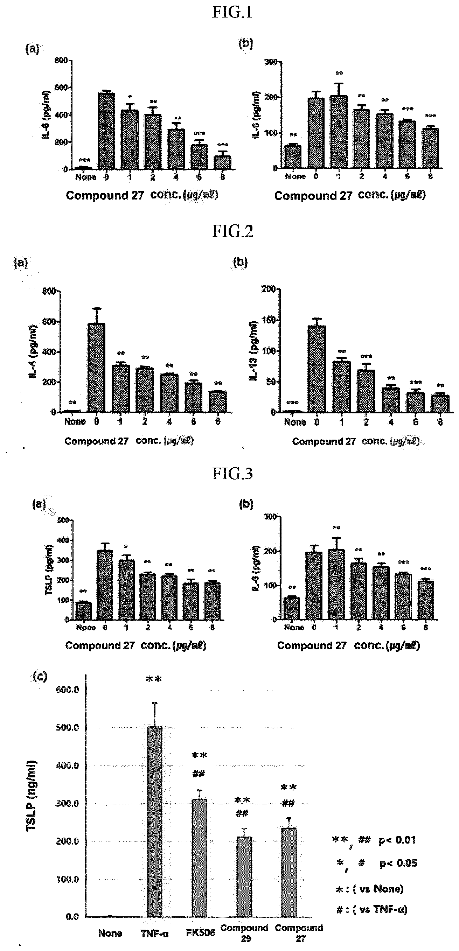

Mouse macrophage RAW264.7 cells (obtained from the Korean Cell Line Bank) were seeded at an initial density of 5 × 10⁵ cells/well in a 12-well plate and cultured at 37°C for 16 to 24 hours. Subsequently, the compounds of the present invention were dissolved in 100% DMSO to achieve concentrations of 0, 1, 2, 4, and 8 µg/mL. The cell culture medium was then treated with the compound samples at each concentration and incubated at 37°C for 1 hour. Lipopolysaccharide (LPS, Sigma-Aldrich, USA) was then added to the final concentration of 1 µg/mL, and the cells were cultured for an additional 24 hours at 37°C to induce interleukin-6. The culture supernatant was collected, and the amount of induced interleukin-6 from RAW264.7 cells was determined using an ELISA kit (LABIS KOMA, South Korea) according to the manufacturer's instructions. The compounds of the present invention exhibited IC50 values within the treatment concentration range (0 to 8 µg/mL), as shown in Tables 1 and 2. In this example, the IC50 value is defined as the concentration of the compound of the present invention that can reduce the level of LPS-induced IL-6 by 50%. Figure 1 (a) illustrates the results for compound 27 of the present invention.

**Table 1**

| Compound Structures |
|---|
| 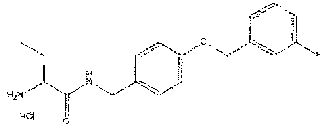 |
| 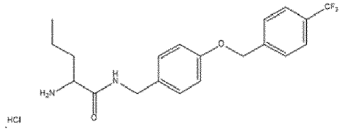 |
| 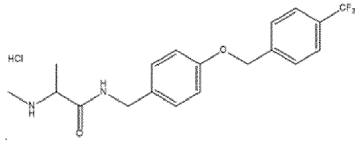 |
| 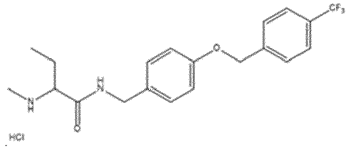 |
| 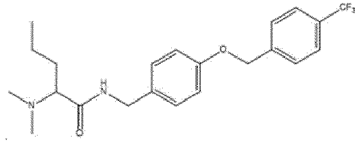 |
| 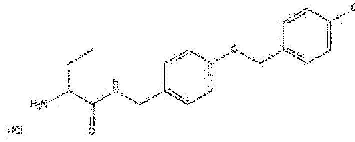 |
| 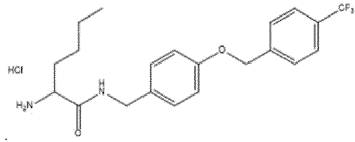 |
| 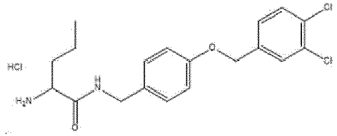 |

**Table 2**

| | | | |
|---|---|---|---|
| 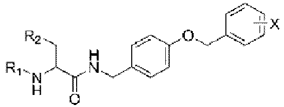 | | | |

| Compound number | R₁ | R₂ | X |
|---|---|---|---|
| 17 | H | CH₃ | 2-F |
| 18 | H | CH₃ | 3-F |
| 19 | H | CH₃ | 4-F |
| 20 | H | CH₃ | 2-CF₃ |
| 21 | H | CH₃ | 3-CF₃ |
| 22 | H | CH₃ | 4-CF₃ |
| 23 | H | CH₃ | 3-Cl |
| 24 | H | CH₃ | 4-Cl |
| **25** | H | CH₃CH₂ | 3-F |
| **26** | H | CH₃CH₂ | 4-CF₃ |
| **27** | CH₃ | CH₃ | 4-CF₃ |
| 28 | CH₃ | CH₃ | 3-Cl |
| 29 | CH₃ | CH₃ | 4-Cl |
| 30 | CH₃ | CH₃CH₂ | 4-CF₃ |
| 31 | (CH₃)₂ | CH₃CH₂ | 4-CF₃ |

Furthermore, human keratinocyte HaCaT cells (obtained from the Korean Cell Line Bank) were seeded at an initial density of 2 × 10⁵ cells/well in a 12-well plate and cultured at 37°C for 16 to 24 hours. The compound 27 of the present invention was then added to the HaCaT cell culture medium at final concentrations of 0, 1, 2, 4, and 8 µg/mL and the cells were cultured at 37°C for 1 hour. Subsequently, to induce interleukin-6, the culture medium was treated with TNF-α (Sigma-Aldrich, USA) and IFN-y (Sigma-Aldrich, USA) to final concentrations of 50 ng/mL each, and the cells were cultured for an additional 24 hours at 37°C. Next, the culture supernatant was collected, and the amount of induced interleukin-6 from HaCaT cells was determined using an ELISA kit (LABIS KOMA, South Korea) according to the manufacturer's instructions. As illustrated in Figure 1 (b), it was confirmed that the concentration of interleukin-6 decreased as the treatment concentration of compound 27 of the present invention increased.

### Example 1-2: Confirmation of Anti-Inflammatory Effect Using Interleukin-4 (IL-4)

Rat basophilic leukemia cells RBL-2H3 (obtained from the Korean Cell Line Bank) were seeded at an initial density of 2 × 10⁵ cells/well in a 48-well plate and cultured at 37°C for 16 to 24 hours. Subsequently, compound 27 of the present invention was added to the RBL-2H3 cell culture medium at final concentrations of 0, 1, 2, 4, and 8 µg/mL and the cells were cultured at 37°C for 1 hour. Next, to induce interleukin-4, the culture medium was treated with PMA (Phosphomolybdic acid, Sigma-Aldrich, USA) and ionomycin (Sigma-Aldrich, USA) to final concentrations of 50 ng/mL and 0.5 µM, respectively, and the cells were cultured for an additional 24 hours at 37°C. The culture supernatant was then collected, and the amount of induced interleukin-4 from RBL-2H3 cells was determined using an ELISA kit (LABIS KOMA, South Korea) according to the manufacturer's instructions. Figure 2 (a) represents the results. As observed in Figure 2 (a), it was confirmed that the amount of interleukin-4 decreased proportionally with the increasing treatment concentration of compound 27 of the present invention.

### Example 1-3: Confirmation of Anti-Inflammatory Effect Using Interleukin-13 (IL-13)

RBL-2H3 cells were seeded at an initial density of 2 × 10⁵ cells/well in a 24-well plate and cultured at 37°C for 16 to 24 hours. Subsequently, compound 27 of the present invention was added to the RBL-2H3 cell culture medium at final concentrations of 0, 1, 2, 4, and 8 µg/mL, and the cells were cultured at 37°C for 1 hour. Next, to induce interleukin-13, the culture medium was treated with PMA (Phosphomolybdic acid, Sigma-Aldrich, USA) and ionomycin (Sigma-Aldrich, USA) to final concentrations of 50 ng/mL and 0.5 µM, respectively, and the cells were cultured for an additional 24 hours at 37°C. The culture supernatant was then collected, and the amount of induced interleukin-13 from RBL-2H3 cells was determined using an ELISA kit (R&D Systems, USA) according to the manufacturer's instructions. Figure 2 (b) represents the results. As observed in Figure 2 (b), it was confirmed that the amount of interleukin-13 decreased proportionally with the increasing treatment concentration of compound 27 of the present invention. This indicates the anti-inflammatory effect of compound 27 of the present invention.

### Example 1-4: Confirmation of Anti-Inflammatory Effect after TSLP Induction in HaCaT Cells

Human keratinocyte HaCaT cells (obtained from the Korean Cell Line Bank) were seeded in a 12-well plate at an initial density of 2 × 10⁵ cells/well and cultured at 37°C for 16 to 24 hours. FK506 (Sigma), compounds 27 and 29 of the present invention were then added to the HaCaT cell culture medium at final concentrations of 0, 1, 2, 4, and 8 µg/mL, and the cells were cultured at 37°C for 1 hour. Next, to induce TSLP, the culture medium was treated with TNF-α (Sigma-Aldrich, USA) to a final concentration of 100 ng/mL, and the cells were cultured for an additional 24 hours at 37°C. The culture supernatant was collected, and the amount of induced TSLP from HaCaT cells was determined using an ELISA kit (R&D Systems, USA) according to the manufacturer's instructions. Figure 3 (a) shows that, as the treatment concentration of compound 27 of the present invention increased, TSLP decreased. Under the same conditions, to compare the TSLP reduction efficiency R.R (Reduction rate) of compound 27, positive control compound and compound 29, equal amounts of compounds 27, 29 and FK506 (Sigma-Aldrich, USA) were pretreated for 1 hour. The amount of TSLP released into the culture supernatant from HaCaT cells stimulated by TNF-α was quantitatively analyzed and presented in Table 3 and Figure 3 (c).

**Table 3**

| Compound | TSLP (ng/mf) | R.R (%) |
|---|---|---|
| None | 2 | 100 |
| TNF-α | 503 | 0 |
| Compound 29 | 210 | 58 |
| Compound 27 | 234 | 54 |

### Example 1-5: Confirmation of Anti-Inflammatory Effect after Interleukin-6 Induction in HaCaT Cells

Human keratinocyte HaCaT cells (obtained from the Korean Cell Line Bank) were seeded in a 12-well plate at an initial density of 2 × 10⁵ cells/well and cultured at 37°C for 16 to 24 hours. Compound 27 of the present invention was then added to the HaCaT cell culture medium at final concentrations of 0, 1, 2, 4, and 8 µg/mL, and the cells were cultured at 37°C for 1 hour. Next, to induce interleukin-6, the culture medium was treated with TNF-α (Sigma-Aldrich, USA) and IFN-y (Sigma-Aldrich, USA) to final concentrations of 50 ng/mL each, and the cells were cultured for an additional 24 hours at 37°C. The culture supernatant was collected, and the amount of induced interleukin-6 from HaCaT cells was determined using an ELISA kit (LABIS KOMA, South Korea) according to the manufacturer's instructions. Figure 3 (b) shows that, as the treatment concentration of compound 27 of the present invention increased, interleukin-6 decreased.

### Example 2: Inhibition of TSLP:TSLPR Interaction and Signaling

### 2-1: Calcium Imaging

Human embryonic kidney cells, HEK293T cells (obtained from the Korean Cell Line Bank), were transfected with expression vectors for mouse TSLPR (mTSLPR, NCBI Reference Sequence: NM_001164735 CDC sequence, SEQ ID NO.: 1) or human TSLPR (hTSLPR, NCBI Reference Sequence NM_022148 CDC sequence, SEQ ID NO.: 2); mouse IL7Rα (mIL7R-alpha, NCBI Reference Sequence NM_008372 CDC sequence, SEQ ID NO.: 3) or human IL-7Rα (hILIL-7Rα, NCBI Reference Sequence NM_002185 CDC sequence, SEQ ID NO.: 4); and mouse TRPA1 (mTRPA1, NCBI Reference Sequence NM_001348288 CDC sequence, SEQ ID NO.: 5) or human TRPA1 (hTRPA1, NCBI Reference Sequence NM_007332 CDC sequence, SEQ ID NO.: 6) using Lipofectamine 3000 (Invitrogen, USA) for efficient overexpression. The cells were pre-treated with various concentrations (10 µM, 1 µM, 100 nM, 10 nM, 5 nM, 1 nM, 100 pM) of the compound of the present invention for 10 minutes. To measure changes in intracellular calcium ions, a calcium-specific dye (Fluo-3, AM, Invitrogen, USA) was used. The cells, which were pre-treated or not with the compound of the present invention, were treated with mouse recombinant TSLP protein (mTSLP, R&D Systems, USA) at 2.5 ng/mL or human recombinant TSLP protein (hTSLP, R&D Systems, USA) at 10 ng/mL, and changes in intracellular calcium ion levels induced by them were analyzed. The changes in intracellular calcium ions in the group treated with the compound of the present invention were compared to the intracellular calcium ion changes in cells that were not pretreated with the compound of the present invention. The signal (F0) by the intracellular calcium-specific staining reagent in the group untreated with the compound of the present invention (F0) was compared with the signal (F) by the intracellular calcium-specific staining reagent in the group treated with the compound of the present invention. FIFO of 1 indicates that intracellular calcium influx is completely inhibited by the compound of the present invention. In this example, it was confirmed that the compound of the present invention inhibits intracellular calcium influx, and the IC50 value, defined as the concentration at which 50% inhibition occurs, was determined. Table 4 below shows the results. A: IC50 value less than 500 nM, B: IC50 value equal to or greater than 500 nM.

**Table 4**

| Compound | assay |
|---|---|
| 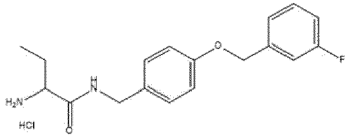 | B |
| 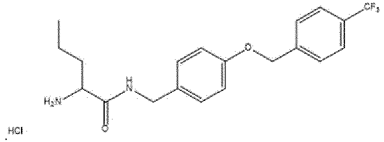 | B |
| 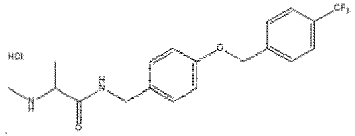 | A |
| 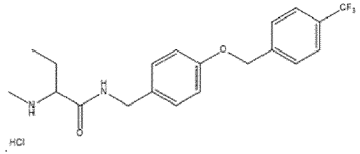 | A |
| 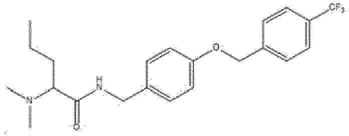 | B |
| 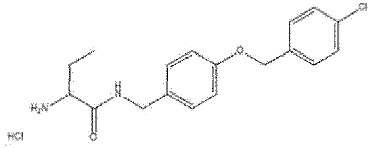 | B |
| 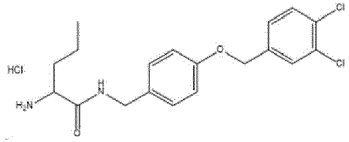 | B |

### 2-2: Inhibition of STATS Phosphorylation

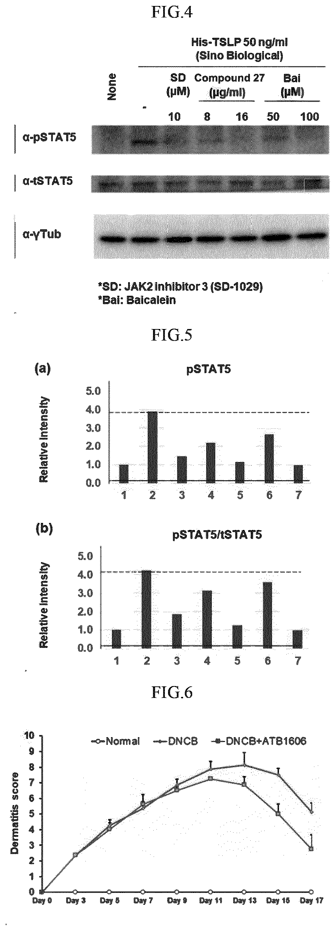

HEK293 cells were seeded at a density of 3 × 10⁵ cells/well in a 12-well plate and cultured overnight (approximately 16 hours). TSLPR/IL-7Ra was introduced into each well at a concentration of 50 ng per well, and the cells were allowed to express for 24 hours. Various concentrations (0, 10, 50, and 100 ng/mL) of His-TSLP were then added to each well. After 0.5 hours, the supernatant and lysate were collected. The lysate and supernatant were mixed with sample buffer and boiled at 99 °C for 5 minutes, followed by storage at -20 °C until Western blotting (WB). The results are shown in Figures 4 and 5. For Western blotting, blocking was performed at room temperature for 2 hours using 5% BSA in TBST as the blocking solution. Primary antibodies, including phospho-STATS, total STAT-5 (Abcam, USA), and gamma Tubulin (Sigma-Aldrich, USA), were used and incubated overnight at 4 °C. The next day, secondary antibodies (anti-rabbit, anti-mouse, SantaCruz, USA) were used, and after incubation at room temperature for 2 hours, the membrane was washed with TBST. Protein bands were detected using the ECL reaction with HRP-conjugated secondary antibodies, and iBright (Thermo Fisher Scientific) equipment was used for visualization. Baicalein, known as a TSLP response inhibitor, and SD-1029 (CAS 118372-34-2, Sigma-Aldrich), a JAK2 inhibitor, were used as controls.

### Example 3: Efficacy Experiment Using DNCB-Induced Atopic Dermatitis Mouse Model Experimental Method:

ICR mice (Orient Bio Inc., male, 4 weeks old) were used, with four mice per group. The mice were acclimatized for one week under controlled conditions (23 ± 2 °C, humidity 55 ± 10%, 12-hour light/dark cycle, animal facility at Sungkyunkwan University) and were shaved using clippers and depilatory cream before drug treatment. Following the conventional method, 1% DNCB (2,4-dinitrochlorobenzene, Sigma-Aldrich Co., St. Louis, USA) (200 µl) was applied to the dorsal area of the mice every 2 days, four times a week for one week. Subsequently, 0.5% DNCB (200 µl) was applied again at 2-day intervals, eight times, to induce atopic dermatitis (Ara, J., Mol Cell Toxicol 2019). One week after DNCB treatment, signs of lesions were confirmed in all mice. Then, the mice were topically administered with the compound 27 of the present invention, dissolved in sterile water at a concentration of 1% (w/v), starting from the point of 0.5% DNCB application, once daily for two weeks. The experimental animals were supplied with powdered feed (Purina, Switzerland), and water and feed were freely available throughout the experimental period. Symptoms associated with atopic dermatitis, such as erythema, edema, scaling, crusting, lichenification, inflammation, bleeding and skin dryness, were observed and recorded according to the Scoring Atopic Dermatitis (SCORAD) index in Table 2.

**Table 5**

| Symptoms | Score |
|---|---|
| Erythema | 0 : none |
| Edema/Hematoma | 1 : Mild |
| Erosion | 2 : Moderate |
| | 3 : Severe |
| Lithification | (maximum : 15) |
| Dryness | |

Using the Mosquito Blood Collection System, blood was drawn from the retro-orbital plexus, and serum was separated. Skin tissue proteins were extracted using a homogenizer in 0.2% PBS (phosphate buffer saline). To measure the concentration of IgE, an IgE ELISA kit (Shibayagi, Japan) was used following the manufacturer's instructions, and analysis was performed using an ELISA reader. The concentrations of TNF-a, IL-4, CCL22/MDC, MMP-12, and S100A9 were measured using Luminex^{®} Assays (R&D Systems, USA).

### Results:

### 3-1: SCORAD Index

Observations were made for erythema, edema, scaling, crusting, lichenification, inflammation, bleeding and skin dryness. The Scoring Atopic Dermatitis (SCORAD) index (refer to Table 5) was recorded. The results showed that in the DNCB-treated group, there was progression in wounds, keratinization, and shedding of keratinized skin cells. However, in the group treated with compound 27 of the present invention, a significant reduction in symptoms of dermatitis was observed. Figure 6 represents the SCOARD index.

### 3-2: Histopathological Analysis

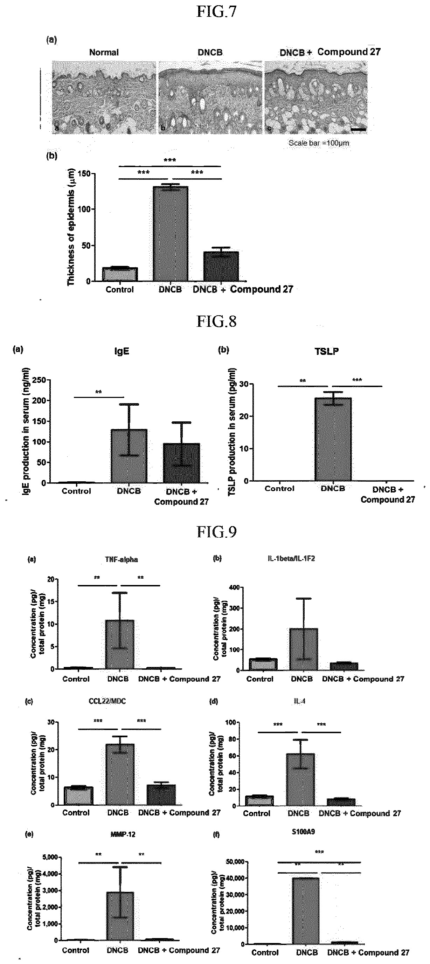

Skin tissues from the mouse dorsal area were separated and fixed in 10% formalin (10% Neutral Buffered Formalin, Kang Dong Trading, South Korea) for 72 hours. The fixed tissues were washed with water for about 1 hour and then processed into paraffin blocks. The paraffin blocks were sectioned into 8 µm slices using a microtome, followed by staining with Harris's hematoxylin solution. Subsequently, the sections were treated with 1% HCl-alcohol, neutralized with 0.5% ammonia solution, and stained with Eosin solution. Next, dehydration was performed using alcohol and xylene, and the sections were mounted on slides using Canada balsam. Figures 7 (a) and (b) represent the results. As shown in Figures 6 and 7, the group treated with compound 27 of the present invention showed a significant decrease in the expression of inflammatory cells compared to the DNCB-treated group. In addition, there was a significant reduction in the thickness of the epidermal layer.

### 3-3: Serum Analysis - IgE and TSLP Expression

To determine the concentrations of IgE and TSLP in mouse serum, IgE ELISA kit (Shibayagi, JP) and TSLP ELISA kit (R&D systems, USA) were used following the manufacturer's instructions. As shown in Figures 8 (a) and (b), the serum concentrations of IgE and TSLP increased in the DNCB-treated group. In contrast, the group treated with compound 27 of the present invention exhibited a decrease in IgE and TSLP concentrations.

### 3-4: Skin Tissue Analysis

Proteins were extracted from skin tissues of atopic dermatitis mice on the dorsal area, and the levels of TNF-α, IL-1β, CCL22/MDC, IL-4, MMP-12, and S100A9 were measured using Luminex^{®} Assays (R&D systems, USA). As depicted in Figures 9 (a) to (f), the expression levels of these cytokines and chemokines significantly increased in the DNCB-treated group. However, in the group treated with compound 27 of the present invention, all of them showed a significant decrease.

### Example 4: Efficacy Experiment Using DNFB-Induced Atopic Dermatitis Mouse Model

### Experimental Procedure:

ICR mice (Supplier: Koatech, Gyeonggi, South Korea, Male, 9 weeks old) were used. After acclimatization to the environment for one week, the mice were divided into treatment and control groups, and then hair was removed from the area (dorsal area) where the drug was applied on day 0, 0.5% DNFB (50 µL) diluted in 99% acetone, was applied to the dorsal area. Subsequently, on days 5, 8, 10 and 14, additional applications of 0.2% DNFB (20 µL) were applied to induce atopic dermatitis. After 7 days of DNFB treatment, once skin lesions were confirmed in all mice, compound 27 of the present invention was administered daily for one week via intraperitoneal (ip), oral (po), or topical (dermal) routes.

### Results:

### Itch Scratching Test:

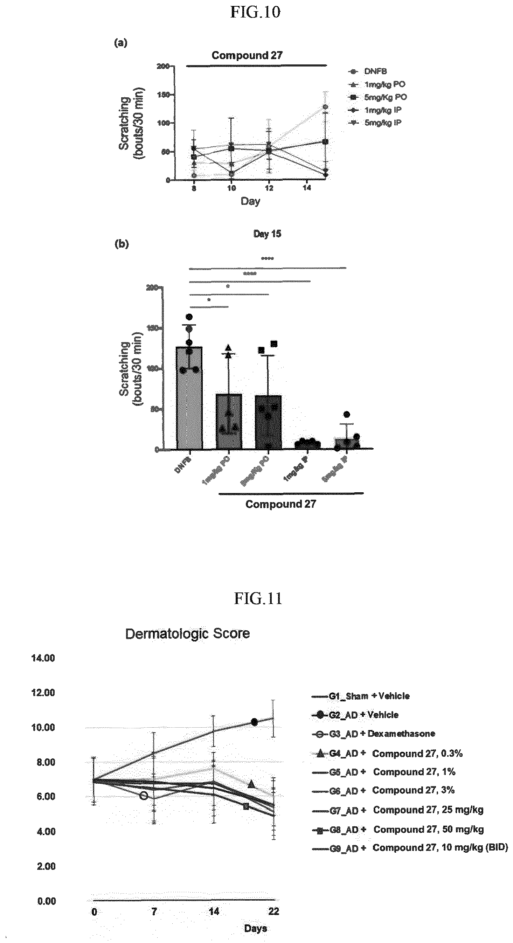

For the groups treated with the compound 27 of the present invention (1 ~ 10 mg/kg) and the control group, mice were separated on days 5, 8, 11 and 15, acclimatized for 1 hour, and their scratching behavior patterns were recorded and analyzed for 30 minutes. The results are shown in Figure 10 (a) and (b). As depicted in Figure 10, the scratching frequency in the compound 27 treatment group significantly decreased compared to the control group.

### Example 5: Efficacy Experiment using House Dust Mite (HDM) Atopic Dermatitis Mouse Model

### Experimental Method:

Nc/Nga mice (Supplier: Central Animal Laboratory, South Korea, Male, 8 weeks old) were used, with 8 mice per group. The experimental animals were allowed free access to Purina Nestle feed (Switzerland) and water. The mice were shaved using clippers and depilatory cream one day (day 0) before applying Df cream (Dermatophagoides farina cream, Biostir AD, Biostir Incorporation). Df cream was applied to the mouse dorsal area twice a week for 5 weeks to induce atopic dermatitis. After confirming lesions in all mice, compound 27 of the present invention was administered topically or orally for 3 weeks (21 days) from 2 weeks after Df cream application. For topical administration, compound 27 was mixed with 100% ethanol to achieve concentrations of 0.3%, 1%, or 3%, and applied once daily. It was applied to both ears (25 µL each) and the dorsal area (70 µL). 100% ethanol was used as the topical vehicle. For oral administration, compound 27 was administered at doses of 25 mpk (once daily, QD), 50 mpk (QD), or 10 mpk (twice daily, BID). The vehicle for oral administration was a mixture of DMSO (Dimethyl sulfoxide): PEG300 (Polyethylene Glycol): H₂O in a ratio of 1:4:5. Dexamethasone (Sigma, Cat no. D1756) was used as a positive control and administered orally at 25 mpk once daily (QD). Dexamethasone, used as a positive control, was prepared using sterile distilled water to achieve the appropriate dose and volume. The composition of the test groups is presented in Table 6 below.

**Table 6**

| Group | Df cream induction | Drug | Dose | Route | Volume | Frequen cy | Duratio n (days) | No. of mice |
|---|---|---|---|---|---|---|---|---|
| G1 | N | Vehicle | - | Oral | 10 mℓ/kg | QD | 21 | 8 |
| G2 | Y | Vehicle | - | Oral | 10 mℓ/kg | QD | 21 | 8 |
| G3 | Y | Dexamethas one | 2.5 mg/kg | Oral | 10 mℓ/kg | QD | 21 | 8 |
| G4 | Y | Compound 27 | 0.3% | Topical | 120 µℓ/head | QD | 21 | 8 |
| G5 | Y | Compound 27 | 1% | Topical | 120 µℓ/head | QD | 21 | 8 |
| G6 | Y | Compound 27 | 3% | Topical | 120 µℓ/head | QD | 21 | 8 |
| G7 | Y | Compound 27 | 25 mg/kg | Oral | 10 mℓ/kg | QD | 21 | 8 |
| G8 | Y | Compound 27 | 50 mg/kg | Oral | 10 mℓ/kg | QD | 21 | 8 |
| G9 | Y | Compound 27 | 10 mg/kg | Oral | 10 mℓ/kg | BID | 21 | 8 |

### Results

### 5-1: Inflammation Score (Dermatitis score)

Skin indicators were recorded through visual observation once a week (on days 0, 7, 14, 21, 28, and 36). The evaluation was conducted using the Modified SCORAD score, with entries for erythema/hemorrhage(or bleeding), edema, excoriation/erosion, and scaling/dryness. Each item was scored as follows: no symptoms (0 points), mild symptoms (1 point), moderate symptoms (2 points), and severe symptoms (3 points). The total score was calculated by summing up scores for all items. As shown in Figure 11, compound 27 of the present invention exhibited a superior effect in improving skin lesions compared to the control group 10 days after administration of the drug in the present invention.

### Example 6: Analysis of Skin-Specific Memory T Cell Markers

### Experimental Method

Nc/Nga mice (purchased from Central Laboratory Animal, male, 6 weeks old) were used, with 2 mice per group. The experimental animals were allowed to freely access to food and water. Mice were shaved using clippers and depilatory cream before the application of Df cream. Df cream (dermatophagoides farina cream) (Biostir AD, Biostir Incorporation) was applied to the dorsal area of the mice 3 times a week for 4 weeks to induce atopic dermatitis. After confirming lesions induced in all mice, compound 27 of the present invention was applied topically to the skin three times a week for three weeks starting four weeks after the Df cream treatment. In contrast to other examples, this example excluded the simultaneous application of the test drug (compound 27 of the present invention) and the atopic dermatitis-inducing agent during the induction period. The compound of the present invention (8 µg/mL and 16 µg/mL) was applied to the skin in a volume of 100 µL. The control and test groups used in this example are listed in Table 7. As a positive control, 0.1% dexamethasone was topically applied to the mouse skin for comparison.

**Table 7**

| Control groups | Experimental groups |
|---|---|
| NC/Nga mouse without HDM | |
| NC/Nga mouse with HDM | NC/Nga mouse with HDM + ATB 1606 8 *µ*g/mL |
| | NC/Nga mouse with HDM + ATB 1606 8 *µ*g/mL |
| NC/Nga mouse with HDM treatment + 0.1% Dexamethasone (Positive control) | |

### Results

### 6-1: Comparison of Inflammatory Response

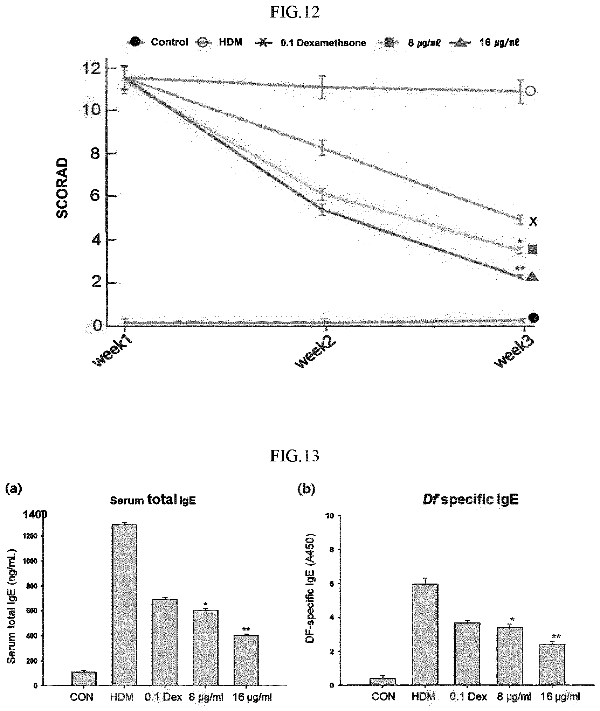

Erythema, hemorrhage, wounds, dryness, swelling, and shedding in mice were observed visually and compared using the SCORAD score. Erythema and hemorrhage were observed in the positive control group treated with 0.1% dexamethasone. In comparison to the positive control group, the experimental group treated with compound 27 of the present invention showed a significant reduction in erythema, hemorrhage, dryness, wounds, swelling, and shedding, indicating complete healing of wounds and erythema. Figure 12 graphically represents the changes in the atopic dermatitis mouse model's skin scores due to the treatment with each test substance (Wilcoxon-signed rank test; P < 0.05 with statistically significant three mice were examined from each group). As observed in Figure 12, compound 27 of the present invention improved atopic dermatitis symptoms in a concentration-dependent manner (16 µg/mL, 8 µg/mL), demonstrating superiority over the positive control group treated with 0.1% dexamethasone.

### 6-2: Serum Analysis

A mouse IgE ELISA Kit (BioLegend, San Diego, CA, USA) and a mouse DF-specific IgE ELISA Kit (BioLegend, San Diego, CA, USA) were used according to the manufacturer's instructions to measure IgE in mouse serum and mouse DF-specific IgE. Changes in the levels of total IgE and DF-specific IgE in the serum were presented in Figure 13 (a) and (b), respectively. In comparison to the positive control group treated with 0.1% dexamethasone, a significant decrease in both total IgE and DF-specific IgE was observed in the compound-treated group of the present invention, as shown in Figure 13.

### 6-3: Tissue Analysis

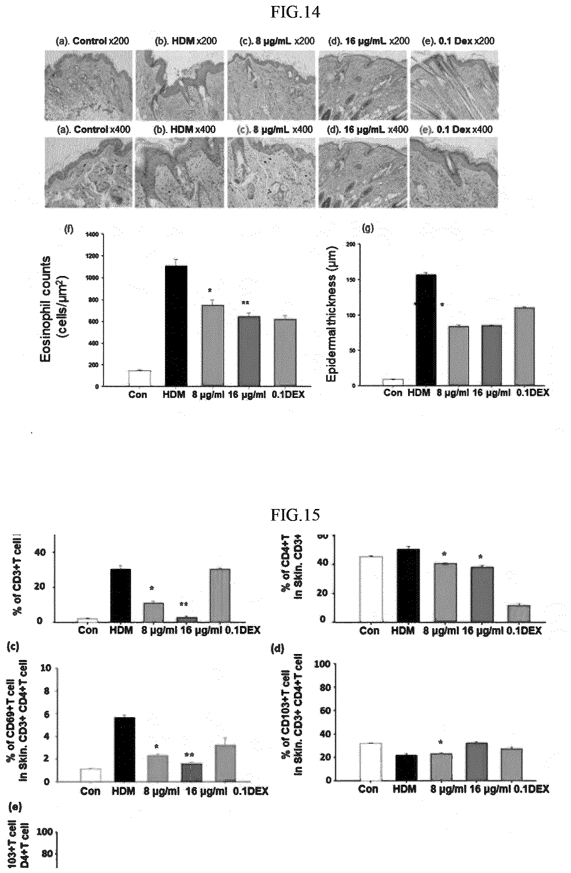

Skin tissue changes were examined using Hematoxylin and Eosin (H&E) staining (refer to Figure 15) (Data are shown as mean ± standard deviation (SD). ** P < 0.01, * P < 0.05). Additionally, changes in eosinophils and mouse epidermal thickness were measured (see Figure 22). In the case of the compound 27-treated group of the present invention, there was a concentration-dependent decrease in eosinophil count, and the epidermal thickness of mice appeared to thin with increasing drug concentration.

### 6-4: FACS Analysis

### (1) Analysis of T Cell Fractions in the Skin of Atopic Dermatitis Mice

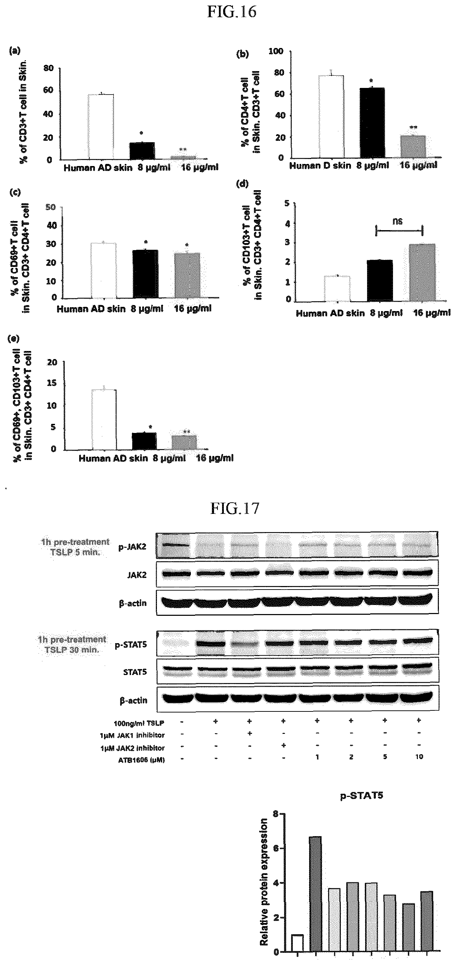

The results of the analysis of T cell fractions in the skin of atopic dermatitis mouse models treated with compound 27 of the present invention are presented in Figures 16 and 17 (data are shown as mean ± standard deviation (SD). ** P < 0.01, * P < 0.05). In both compound 27-treated groups (16 µg/mL, 8 µg/mL), there was a concentration-dependent significant decrease in the proportion of skin-specific T cells, such as CD3+, CD4+, CD69+, and CD103+. In particular, the numbers of CD69+ T cells and CD103+ T cells showed a statistically significant decrease, demonstrating a markedly superior effect compared to the positive control group treated with 0.1% dexamethasone.

### (2) Analysis of Skin Samples from Atopic Dermatitis Patients

For tissues isolated from the skin of atopic dermatitis patients (Yonsei Severance Hospital, South Korea) followed by the treatment of the compound 27 of the present invention, T cell fractions were compared, and the results are presented in Figures 18 and 19 (Data are shown as mean ± standard deviation (SD). ** P < 0.01, * P < 0.05). In both compound 27-treated groups (16 µg/mL, 8 µg/mL), there was a concentration-dependent significant decrease in the proportion of skin-specific T cells, demonstrating a remarkable reduction in both CD69+ T cells and CD103+ T cells.

### Example 7: Confirmation of Signaling Pathway Regulation Using Bone Marrow-Derived Dendritic Cells (BMDCs)

### 7-1. Cell Culture

C57BL/6J mice (JA BIO, DBL-0011, male, 6-12 weeks old) were euthanized, and femurs and tibias were separated after cervical dislocation and then were stored in PBS (HyClone, Cat No. SH30028.02). After removing tissues as much as possible from femurs and tibias, both ends of the bones were cut vertically, and bone marrow was quickly collected with a 5 mL syringe (Korea Vaccine, 5cc syringe 22g). After centrifugation at 1800 rpm for 3 minutes, the supernatant was discarded, 10 mL PBS was added to resuspend the cells, and the mixture was filtered through a 100 µm cell strainer. Following additional centrifugation at 1800 rpm for 3 minutes, the supernatant was discarded, and the cells were cultured in culture media [RPMI 1640 (Corning, Cat No. 10-040-CV), 10% fetal bovine serum (FBS, Gibco, Cat No. 26140-079), 1% Penicillin-Streptomycin (Gibco, Cat No. 15140-122), 50 µM 2-Mercaptoethanol (Gibco, Cat No. 21985), 20 µg/mL GM-CSF (Peprotech, Cat No. 315-03)] in a CO₂ incubator. After 4 days, an equal volume of culture media was added, and the cells were cultured for an additional 2 days. Bone marrow-derived dendritic cells (BMDCs) were then collected, seeded at a concentration of 8 × 10⁵ cells/mL in a 12-well plate, and incubated overnight.

### 7-2. Drug and TSLP Treatment

The obtained BMDCs were pre-treated with compound 27, Jak1 inhibitor (Filgotinib, Selleck, Cat No. JS7605), or Jak2 inhibitor (SD-1029, Sigma, Cat No. 573098) 30 minutes or 1 hour prior to treatment. Subsequently, mTSLP (R&D Systems, Cat No. 555-TS-010) or hTSLP was applied, and after 5, 10, or 30 minutes, protein lysates were obtained using lysis buffer (Cell signaling, 9806S). The lysates were centrifuged at 15,000 rpm for 20 minutes at 4°C, the supernatant was collected, and transferred to a new E-tube. After quantifying the protein using the BCA protein assay kit (Thermo, Cat No. 23225), 5X SDS-PAGE loading buffer (Biosesang, Cat No. SF2002-110-00) was added, boiled for 5 minutes at 103°C, and stored at -20°C.

### 7-3: Western Blot

SDS-PAGE gels were prepared with different compositions depending on the molecular weight of the proteins, assembled in a BIO-RAD cassette, loaded with the sample following adding running buffer, and run at 60 to 100 V for a specific duration. The samples were to a PVDF membrane (Amersham, Cat No. 10600022) treated with methanol at 100 V for 2 hours. After blocking with 5% BSA (Bovogen, Cat No. BSAS0.1) for 2 hours, the membrane was incubated overnight at 4°C with primary antibodies [phospho-Stat5 antibody (Cell signaling, Cat No. 9351S), total Stat5 antibody (Cell signaling, Cat No. 94205S), γ-Tubulin antibody (Invitrogen, Cat No. T6557), actin (Santa Cruz, Cat No. sc-47778)]. The next day, after washing with TBS-T, the membrane was incubated with HRP-conjugated secondary antibodies [Anti-Rabbit IgG (BIO-RAD, Cat No. BR1706515), Anti-Mouse IgG (BIO-RAD, Cat No. BR1706516)] for 2 hours at room temperature. After washing with TBS-T, the membrane was incubated with ECL solution (Thermo, Cat No. 32209), and protein bands were detected using the iBright imaging system (Invitrogen, Model No. FL1500). Each band intensity was measured using ImageJ software (version 1.38; National Institutes of Health), and the pSTAT5/STAT ratio was calculated. The results were graphically represented using GraphPad Prism software (version 5.01; GraphPad Software, Inc.). As shown in Figure 17, p-STAT5 increased by 6.7-fold with TSLP treatment, while treatment with compound 27 of the present invention significantly reduced p-STAT5. Compared to the TSLP-treated group, treatment with 1 µM of the compound of the present invention showed a 40% reduction in p-STAT5, comparable to the JAK2 inhibitor, and treatment with 2 µM and 5 µM showed 51% and 59% reduction, respectively.

## Claims

1. A pharmaceutical composition for preventing or treating inflammatory disease, comprising a benzyloxybenzylamine amino acid derivative represented by the following Formula 1, or salt, tautomer, polymorph, prodrug, solvate or R-form, S-form or racemic mixture thereof, as a pharmaceutically active ingredient,
wherein the composition regulates intracellular signaling pathway mediated by cytokine thymic stromal lymphopoietin (TSLP), TSLP receptor (TSLPR), and IL-7Rα: 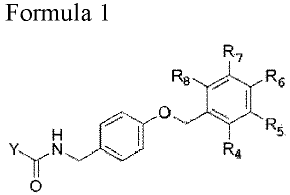
in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formula 2 to 4, 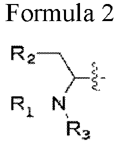 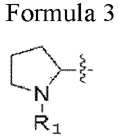 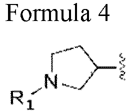
wherein R₁ and R₃ are the same or different from each other, and are each independently hydrogen or C₁₋₇ alkyl, and
R₂ is C₁₋₇ alkyl.

2. The pharmaceutical composition of claim 1, wherein said C₁₋₇ alkyl is straight, branched, or cyclic C₁₋₇ alkyl.

3. The pharmaceutical composition of claim 1, wherein the compound represented by the Formula 1 is any one selected from the group consisting of the following compounds:
*(R)*/*(S)*-1-((4-((2-(fluorobenzyl)oxy)benzyl)amino)-1-oxobutane-2-aminium chloride (Compound 17);
*(R)*/*(S)*-1-((4-((3-(fluorobenzyl)oxy)benzyl)amino)-1-oxobutane-2-aminium chloride (Compound 18);
*(R)*/*(S)*-1-((4-((4-(fluorobenzyl)oxy)benzyl)amino)-1-oxobutane-2-aminium chloride (Compound 19);
*(R)*/*(S)*-1-oxo-1-((4-((2-(trifluoromethyl)benzyl)oxy)benzyl)amino)butane-2-aminium chloride (Compound 20);
*(R)*/*(S)*-1-oxo-1-((4-((3-(trifluoromethyl)benzyl)oxy)benzyl)amino)butane-2-aminium chloride (Compound 21);
*(R)*/*(S)*-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)*-1-((4-((3-fluorobenzyl)oxy)benzyl)amino)-1-oxopentane-2-aminium chloride (Compound 25);
*(R)*/*(S)*-1-((4-((4-(trifluorobenzyl)oxy)benzyl)amino)-1-oxopentane-2-aminium chloride (Compound 26);
*(R)*/*(S)*-2-(methylamino)-N-(4-((4-trifluoromethyl)benzyl)oxy)benzyl)butanamide (Compound 27);
*(R)*/*(S)*-N-(4-((3-chlorobenzyl)oxy)benzyl)-2-(methylamino)butanamide (Compound 28);
*(R)*/*(S)*-N-(4-((4-chlorobenzyl)oxy)benzyl)-2-(methylamino)butanamide (Compound 29);
*(R)*/*(S)*-2-(methylamino)-N-(4-((4-trifluoromethyl)benzyl)oxy)benzyl)pentanamide (Compound 30);
*(R)*/*(S)*-2-(methylamino)-N-(4-((4-trifluoromethyl)benzyl)oxy)benzyl)pentanamide (Compound 31);
*(R)*/*(S)*-tert-butyl-2-((4-((3-fluorobenzyl)oxy)benzyl)carbamoyl)pyrrolidine-1-carboxylate (Compound 34);
*(R)*/*(S)*-tert-butyl-2-((4-((4-fluorobenzyl)oxy)benzyl)carbamoyl)pyrrolidine-1-carboxylate (Compound 35);
*(R)*/*(S)*-tert-butyl-2-((4-((4-(trifluoromethyl)benzyl)oxy)benzyl)carbamoyl)pyrrolidine-1-carboxylate (Compound 36);
*(R)*/*(S)*-tert-butyl-2-((4-((4-chlorobenzyl)oxy)benzyl)carbamoyl)pyrrolidine-1-carboxylate (Compound 37);
*(R)*/*(S)*-N-(4-((3-fluorobenzyl)oxy)benzyl)pyrrolidine-2-carboxamide hydrochloride (Compound 38)
*(R)*/*(S)*-N-(4-((4-fluorobenzyl)oxy)benzyl)pyrrolidine-2-carboxamide hydrochloride (Compound 39);
*(R)*/*(S)*-N-(4-((4-(trifluoromethyl)benzyl)oxy)benzyl)pyrrolidine-2-carboxamide hydrochloride (Compound 40);
*(R)*/*(S)*-N-(4-((4-chlorobenzyl)oxy)benzyl)pyrrolidine-2-carboxamide hydrochloride (Compound 41);
*(R)*/*(S)*-1-methyl-N-(4-((3-fluorobenzyl)oxy)benzyl)pyrrolidine-2-carboxamide (Compound 42);
*(R)*/*(S)*-1-methyl-N-(4-((4-(trifluoromethyl)benzyl)oxy)benzyl)pyrrolidine-2-carboxamide (Compound 43);
*(R)*/*(S)*-1-methyl-N-(4-((4-chlorobenzyl)oxy)benzyl)pyrrolidine-2-carboxamide (Compound 44);
*(R)*/*(S)*-tert-butyl-3-((4((3-fluorobenzyl)oxy)benzyl)carbamoyl)pyrrolidine-1-carboxylate (Compound 46);
*(R)*/*(S)*-tert-butyl-3-((4((4-(trifluoromethyl)benzyl)oxy)benzyl)carbamoyl)pyrrolidine-1-carboxylate (Compound 47);
*(R)*/*(S)*-tert-butyl-3-((4((4-chlorobenzyl)oxy)benzyl)carbamoyl)pyrrolidine-1-carboxylate (Compound 48);
*(R)*/*(S)-*2-((4-((3-fluorobenzyl)oxy)benzyl)carbamoyl)pyrrolidi-1-nium chloride (Compound 49);
*(R)*/*(S)*-2-((4-((4-(trifluoromethyl)benzyl)oxy)benzyl)carbamoyl)pyrrolidi-1-nium chloride (Compound 50);
*(R)*/*(S)*-2-((4-((4-chlorobenzyl)oxy)benzyl)carbamoyl)pyrrolidi-1-nium chloride (Compound 51);
*(R)*/*(S)*-1-methyl-N-(4-((3-fluorobenzyl)oxy)benzyl)pyrrolidine-3-carboxamide (Compound 52);
*(R)*/*(S)*-1-methyl-N-(4-((4-(trifluoromethyl)benzyl)oxy)benzyl)pyrrolidine-3-carboxamide (Compound 53);
*(R)*/*(S)-*1-methyl-N-(4-((4-chlorobenzyl)oxy)benzyl)pyrrolidine-3-carboxamide (Compound 54);
*(R)*/*(S)-*tert-butyl(1-((4-((3,4-chlorobenzyl)oxy)benzyl)amino)-1-oxobuta-2-nyl)carbamate (Compound 55);
*(R)*/*(S)*-tert-butyl(1-oxo-1-((4-((4-(trifluoromethyl)benzyl)oxy)benzyl)amino)penta-2-nyl)carbamate (Compound 56);
*(R)*/*(S)*-1-((4-((3,4-dichlorobenzyl)oxy)benzyl)amino)-1-oxobutane-2-aminium chloride (Compound 57);
*(R)*/*(S)*-1-((4-((3,4-dichlorobenzyl)oxy)benzyl)amino)-1-oxopentane-2-aminium chloride (Compound 58);
*(R)*/*(S)*-2-(dimethylamino)-N-(4-((4-trifluoromethyl)benzyl)oxy)benzyl)butanamide (Compound 59);
*(R)*/*(S)*-N-(4-((3,4-dichlorobenzyl)oxy)benzyl)-2-(dimethylamino)butanamide (Compound 60);
*(R)*/*(S)*-N-(4-((3,4-dichlorobenzyl)oxy)benzyl)-2-(dimethylamino)butanamide (Compound 61);
*(R)*/*(S)*-2-(ethylamino)-N-(4-((4-(trifluoromethyl)benzyl)oxy)benzyl)butanamide (Compound 62);
*(R)*/*(S)*-N-(4-((3,4-dichlorobenzyl)oxy)benzyl-2-(ethylamino)butanamide (Compound 63);
*(R)*/*(S)*-2-(ethylamino)-N-(4-((4-(trifluoromethyl)benzyl)oxy)benzyl)pentanamide (Compound 64);
*(R)*/*(S)*-N-(4-((3,4-dichlorobenzyl)oxy)benzyl-2-(ethylamino)pentanamide (Compound 65);
*(R)*/*(S)*-2-(diethylamino)-N-(4-((4-(trifluoromethyl)benzyl)oxy)benzyl)butanamide (Compound 66);
*(R)*/*(S)*-N-(4-((3,4-dichlorobenzyl)oxy)benzyl-2-(diethylamino)butanamide (Compound 67);
*(R)*/*(S)*-2-(diethylamino)-N-(4-((4-(trifluoromethyl)benzyl)oxy)benzyl)pentanamide (Compound 68); and
*(R)*/*(S)*-N-(4-((3,4-dichlorobenzyl)oxy)benzyl-2-(diethylamino)pentanamide (Compound 69).

4. The pharmaceutical composition of any one of claims 1 to 3, wherein the inflammatory disease is atopic dermatitis, psoriasis, asthma, rhinitis, rheumatoid arthritis, osteoarthritis, inflammatory bowel disease, or allergic diseases.

5. A pharmaceutical composition for preventing or treating cancer, comprising a benzyloxybenzylamine amino acid derivative represented by the following Formula 1, or salt, tautomer, polymorph, prodrug, solvate or R-form, S-form or racemic mixture thereof, as a pharmaceutically active ingredient,
wherein said composition regulates intracellular signaling pathway mediated by cytokine thymic stromal lymphopoietin (TSLP), TSLP receptor (TSLPR), and IL-7Rα: 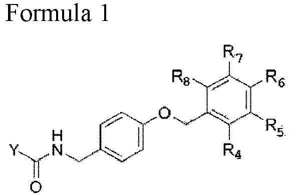
in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formula 2 to 4, 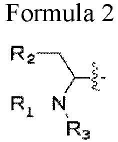 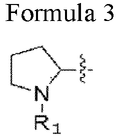 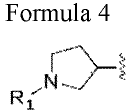
wherein R₁ and R₃ are the same or different from each other, and are each independently hydrogen or C₁₋₇ alkyl, and
R₂ is C₁₋₇ alkyl.

6. The pharmaceutical composition of claim 5, wherein said C₁₋₇ alkyl is straight, branched, or cyclic C₁₋₇ alkyl.

7. The pharmaceutical according to claim 5, wherein the compound represented by the Formula 1 is any one selected from the group consisting of the following compounds:
*(R)*/*(S)*-1-((4-((2-(fluorobenzyl)oxy)benzyl)amino)-1-oxobutane-2-aminium chloride (Compound 17);
(*R*)/(*S*)-1-((4-((3-(fluorobenzyl)oxy)benzyl)amino)-1-oxobutane-2-aminium chloride (Compound 18);
(*R*)/(*S*)-1-((4-((4-(fluorobenzyl)oxy)benzyl)amino)-1-oxobutane-2-aminium chloride (Compound 19);
*(R)*/*(S)*-1-oxo-1-((4-((2-(trifluoromethyl)benzyl)oxy)benzyl)amino)butane-2-aminium chloride (Compound 20);
*(R)*/*(S)*-1-oxo-1-((4-((3-(trifluoromethyl)benzyl)oxy)benzyl)amino)butane-2-aminium chloride (Compound 21);
*(R)*/*(S)*-1-oxo-1-((4-((4-(trifluoromethyl)benzyl)oxy)benzyl)amino)butane-2-aminium chloride (Compound 22);
*(R)*/*(S)*-1-((4-((3-(chlorobenzyl)oxy)benzyl)amino)-1-oxobutane-2-aminium chloride (Compound 23);
*(R)*/*(S)*-1-((4-((4-(chlorobenzyl)oxy)benzyl)amino)-1-oxobutane-2-aminium chloride (Compound 24);
*(R)*/*(S)*-1-((4-((3-fluorobenzyl)oxy)benzyl)amino)-1-oxopentane-2-aminium chloride (Compound 25);
*(R)*/*(S)*-1-((4-((4-(trifluorobenzyl)oxy)benzyl)amino)-1-oxopentane-2-aminium chloride (Compound 26);
*(R)*/*(S)*-2-(methylamino)-N-(4-((4-trifluoromethyl)benzyl)oxy)benzyl)butanamide (Compound 27);
*(R)*/*(S)*-N-(4-((3-chlorobenzyl)oxy)benzyl)-2-(methylamino)butanamide (Compound 28);
*(R)*/*(S)*-N-(4-((4-chlorobenzyl)oxy)benzyl)-2-(methylamino)butanamide (Compound 29);
*(R)*/*(S)-*2-(methylamino)-N-(4-((4-trifluoromethyl)benzyl)oxy)benzyl)pentanamide (Compound 30);
*(R)*/*(S)*-2-(methylamino)-N-(4-((4-trifluoromethyl)benzyl)oxy)benzyl)pentanamide (Compound 31);
*(R)*/*(S)*-tert-butyl-2-((4-((3-fluorobenzyl)oxy)benzyl)carbamoyl)pyrrolidine-1-carboxylate (Compound 34);
*(R)*/*(S)*-tert-butyl-2-((4-((4-fluorobenzyl)oxy)benzyl)carbamoyl)pyrrolidine-1-carboxylate (Compound 35);
*(R)*/*(S)*-tert-butyl-2-((4-((4-(trifluoromethyl)benzyl)oxy)benzyl)carbamoyl)pyrrolidine-1-carboxylate (Compound 36);
*(R)*/*(S)*-tert-butyl-2-((4-((4-chlorobenzyl)oxy)benzyl)carbamoyl)pyrrolidine-1-carboxylate (Compound 37);
*(R)*/*(S)*-N-(4-((3-fluorobenzyl)oxy)benzyl)pyrrolidine-2-carboxamide hydrochloride (Compound 38)
*(R)*/*(S)*-N-(4-((4-fluorobenzyl)oxy)benzyl)pyrrolidine-2-carboxamide hydrochloride (Compound 39);
*(R)*/*(S)*-N-(4-((4-(trifluoromethyl)benzyl)oxy)benzyl)pyrrolidine-2-carboxamide hydrochloride (Compound 40);
*(R)*/*(S)*-N-(4-((4-chlorobenzyl)oxy)benzyl)pyrrolidine-2-carboxamide hydrochloride (Compound 41);
*(R)*/*(S)*-1-methyl-N-(4-((3-fluorobenzyl)oxy)benzyl)pyrrolidine-2-carboxamide (Compound 42);
*(R)*/*(S)*-1-methyl-N-(4-((4-(trifluoromethyl)benzyl)oxy)benzyl)pyrrolidine-2-carboxamide (Compound 43);
*(R)*/*(S)*-1-methyl-N-(4-((4-chlorobenzyl)oxy)benzyl)pyrrolidine-2-carboxamide (Compound 44);
*(R)*/*(S)-*tert-butyl-3-((4((3-fluorobenzyl)oxy)benzyl)carbamoyl)pyrrolidine-1-carboxylate (Compound 46);
*(R)*/*(S)*-tert-butyl-3-((4((4-(trifluoromethyl)benzyl)oxy)benzyl)carbamoyl)pyrrolidine-1-carboxylate (Compound 47);
*(R)*/*(S)*-tert-butyl-3-((4((4-chlorobenzyl)oxy)benzyl)carbamoyl)pyrrolidine-1-carboxylate (Compound 48);
*(R)*/*(S)*-2-((4-((3-fluorobenzyl)oxy)benzyl)carbamoyl)pyrrolidi-1-nium chloride (Compound 49);
*(R)*/*(S)*-2-((4-((4-(trifluoromethyl)benzyl)oxy)benzyl)carbamoyl)pyrrolidi-1-nium chloride (Compound 50);
*(R)*/*(S)*-2-((4-((4-chlorobenzyl)oxy)benzyl)carbamoyl)pyrrolidi-1-nium chloride (Compound 51);
*(R)*/*(S)*-1-methyl-N-(4-((3-fluorobenzyl)oxy)benzyl)pyrrolidine-3-carboxamide (Compound 52);
*(R)*/*(S)*-1-methyl-N-(4-((4-(trifluoromethyl)benzyl)oxy)benzyl)pyrrolidine-3-carboxamide (Compound 53);
*(R)*/*(S)*-1-methyl-N-(4-((4-chlorobenzyl)oxy)benzyl)pyrrolidine-3-carboxamide (Compound 54);
*(R)*/*(S)*-tert-butyl(1-((4-((3,4-chlorobenzyl)oxy)benzyl)amino)-1-oxobuta-2-nyl)carbamate (Compound 55);
*(R)*/*(S)*-tert-butyl(1-oxo-1-((4-((4-(trifluoromethyl)benzyl)oxy)benzyl)amino)penta-2-nyl)carbamate (Compound 56);
*(R)*/*(S)*-1-((4-((3,4-dichlorobenzyl)oxy)benzyl)amino)-1-oxobutane-2-aminium chloride (Compound 57);
*(R)*/*(S)*-1-((4-((3,4-dichlorobenzyl)oxy)benzyl)amino)-1-oxopentane-2-aminium chloride (Compound 58);
*(R)*/*(S)*-2-(dimethylamino)-N-(4-((4-trifluoromethyl)benzyl)oxy)benzyl)butanamide (Compound 59);
*(R)*/*(S)*-N-(4-((3,4-dichlorobenzyl)oxy)benzyl)-2-(dimethylamino)butanamide (Compound 60);
*(R)*/*(S)*-N-(4-((3,4-dichlorobenzyl)oxy)benzyl)-2-(dimethylamino)butanamide (Compound 61);
*(R)*/*(S)*-2-(ethylamino)-N-(4-((4-(trifluoromethyl)benzyl)oxy)benzyl)butanamide (Compound 62);
*(R)*/*(S)*-N-(4-((3,4-dichlorobenzyl)oxy)benzyl-2-(ethylamino)butanamide (Compound 63);
*(R)*/*(S)*-2-(ethylamino)-N-(4-((4-(trifluoromethyl)benzyl)oxy)benzyl)pentanamide (Compound 64);
*(R)*/*(S)*-N-(4-((3,4-dichlorobenzyl)oxy)benzyl-2-(ethylamino)pentanamide (Compound 65);
*(R)*/*(S)*-2-(diethylamino)-N-(4-((4-(trifluoromethyl)benzyl)oxy)benzyl)butanamide (Compound 66);
*(R)*/*(S)*-N-(4-((3,4-dichlorobenzyl)oxy)benzyl-2-(diethylamino)butanamide (Compound 67);
*(R)*/*(S)*-2-(diethylamino)-N-(4-((4-(trifluoromethyl)benzyl)oxy)benzyl)pentanamide (Compound 68); and
*(R)*/*(S)*-N-(4-((3,4-dichlorobenzyl)oxy)benzyl-2-(diethylamino)pentanamide (Compound 69).

8. The pharmaceutical composition of any one of claims 5 to 7, wherein the cancer is pancreatic cancer, breast cancer, skin cancer, oropharyngeal cancer, or gastric cancer.

9. A pharmaceutical composition for preventing or treating allergic diseases, itching or Th2 immune-mediated disorders, comprising a benzyloxybenzylamine amino acid derivative represented by the following Formula 1, or salt, tautomer, polymorph, prodrug, solvate or R-form, S-form or racemic mixture thereof, as a pharmaceutically active ingredient,
wherein the composition regulates intracellular signaling pathway mediated by cytokine thymic stromal lymphopoietin (TSLP), TSLP receptor (TSLPR), and IL-7Rα: 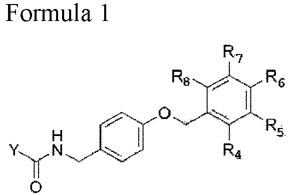
in the Formula 1,
R₄, R₅, R₆, R₇, and R₈ are the same or different from one another, and are each independently selected from hydrogen, C₁₋₇ alkyl, hydroxy, halogen, halogenated C₁₋₇ alkyl, C₁₋₇ alkyloxy, and halogenated C₁₋₇ alkyloxy, and
Y is selected from the following Formula 2 to 4, 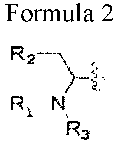 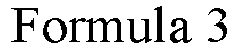 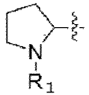 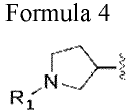
wherein R₁ and R₃ are the same or different from each other, and are each independently hydrogen or C₁₋₇ alkyl, and
R₂ is C₁₋₇ alkyl.
